(19) Europäisches Patentamt
European Patent Office
Office européen des brevets

(11) **EP 4 368 196 A1**

(12) **EUROPEAN PATENT APPLICATION**
published in accordance with Art. 153(4) EPC

(43) Date of publication:
**15.05.2024 Bulletin 2024/20**

(21) Application number: **22838030.9**

(22) Date of filing: **07.07.2022**

(51) International Patent Classification (IPC):
*A61K 38/18* (2006.01)    *A61P 25/30* (2006.01)
*A61P 25/18* (2006.01)    *A61P 25/24* (2006.01)
*A23L 33/17* (2016.01)    *C12Q 1/6883* (2018.01)
*G01N 33/68* (2006.01)    *G01N 33/74* (2006.01)
*A61B 5/00* (2006.01)

(52) Cooperative Patent Classification (CPC):
**A23L 33/17; A61B 5/00; A61K 38/18; A61P 25/18; A61P 25/24; A61P 25/30; C12Q 1/6883; G01N 33/68; G01N 33/74**

(86) International application number:
**PCT/KR2022/009893**

(87) International publication number:
**WO 2023/282678 (12.01.2023 Gazette 2023/02)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **07.07.2021 KR 20210089195**

(71) Applicant: **Adel Inc.**
**Seoul 05505 (KR)**

(72) Inventor: **PARK, Ji Seon**
**Seoul 05505 (KR)**

(74) Representative: **Dehns**
**St. Bride's House**
**10 Salisbury Square**
**London EC4Y 8JD (GB)**

Remarks:
The complete document including Reference Table(s) and the Sequence Listing(s) can be downloaded from the EPO website

(54) **ANIMAL MODEL FOR DISEASES ASSOCIATED WITH ABSENCE OR REDUCTION OF SOCIAL DOMINANCE, AND PHARMACEUTICAL COMPOSITION FOR PREVENTING OR TREATING DISEASES**

(57) The present invention relates to an animal model in which social dominance is absent, and a pharmaceutical composition for preventing or treating diseases associated with the absence or reduction of social dominance. In an animal model, according to the present invention, social dominance is absent through inhibiting of the expression or activity of a CREB-regulated transcription coactivator 3 (CRTC3) gene or protein, and the animal model can be used as a model for diseases associated with the absence or reduction of social dominance. In addition, the present invention enables the prevention, alleviation or treatment of diseases associated with the absence or reduction of social dominance by using a composition containing agonists for brain epidermal growth factor (EGF) receptors.

[FIG. 7A]

WT, CRTC3 KO group-AREG infusion

EP 4 368 196 A1

[FIG. 7B]

Warm spot

[FIG. 7C]

C57BL/6 group - AREG infusion

[FIG. 7D]

C57BL/6 group - AREG-EGF infusion

[FIG. 7E]

WT, CRTC3 KO group - AREG-EGF infusion

[FIG. 7F]

C57BL/6 group - AREG-HB infusion

C57BL/6 group - mEGF infusion

2

## Description

### Technical Field

The present disclosure relates to an animal model lacking social dominance and a composition for preventing or treating a disease or condition related to lack or reduction of social dominance. More specifically, the present disclosure relates to an animal model lacking social dominance, in which expression or activity of CREB-regulated transcription coactivator 3 (CRTC3) gene or protein is suppressed, and a pharmaceutical composition for preventing or treating a disease related to lack or reduction of social dominance, the composition comprising as an active ingredient an agonist for epidermal growth factor (EGF) receptor in the brain.

This application claims priority based on Korea Patent Application No. 10-2021-0089195 filed on July 7, 2021, the entire disclosure of which is incorporated herein by reference.

### Background Art

cAMP-responsive element binding protein (CREB) is expressed in various cells and is well known to activate several eukaryotic transcription factors. In particular, CREB is an important intracellular protein that regulates expression of genes of neurotransmitters and hormones related to social dominance in various types of brain cells, and has been studied as a target for treatment of depressive disorder. In a case where CREB is phosphorylated, the CBP/P300 coactivator that binds to CREB is recruited to activate cAMP response element (CRE)-mediated gene transcription. Recently, the CREB-regulated transcription coactivator (CRTC; transducer of regulated CREB activity, TORC), which is a new CREB coactivator, has been studied. Under basal conditions, CRTC binds to 14-3-3 protein and exists in a phosphorylated state in the cytoplasm. Increases in cAMP and calcium induce dephosphorylation of CRTC and release of 14-3-3 protein. Dephosphorylated CRTC translocates to the nucleus and binds to the bZIP DNA domain of CREB, thereby activating CRE-mediated gene transcription.

CRTC expressed in mammalian tissue consists of three isoforms (CRTC1, CRTC2, and CRTC3). CRTC1 is mainly produced in the brain and is involved in memory, behavior, circadian clock, and energy expenditure. In addition, CRTC2 is expressed in the brain and pancreatic islets where hormone homeostasis is regulated. CRTC2 and CRTC3 are found in peripheral tissue, liver, fat, and bone marrow-derived immune cells. CRTC2 maintains glucose homeostasis in the liver, while CRTC3 is associated with energy balance of adipose tissue and inflammation regulation function of macrophages. However, no research has been done on how CRTC3 works in the brain. Therefore, there is a great need to study whether CRTC3 is expressed in the brain and what role it plays. In particular, it is necessary to find out whether CRTC3 also affects gene expression in addition to CREB activation.

Meanwhile, animal and human societies are closely related to social hierarchy. Human society and many other societies require skills that require social interaction to maintain social hierarchy and achieve communication. In these social relationships, social dominance is a behavior often observed in many social species among all animal systems. In most animal societies, individuals become relatively dominant or subordinate to other individuals. The social stratification formed in this way has a significant impact on health and well-being. In other words, social status and dominance are known to be important factors that determine the degree of survival, health, and reproductive success. In particular, it has been reported that this social dominance is linked to a person's psychological state and may be associated with various psychiatric disease conditions. Specifically, it has been reported that social dominance may be associated with conditions of drug intoxication (Non-Patent Document 1), schizophrenia (Non-Patent Documents 2 and 3), depressive disorder (Non-Patent Document 4), fragile X syndrome (Non-Patent Document 5), autism spectrum disorder and autism (Non-Patent Documents 2 and 6).

With advancements in neuroimaging and molecular techniques in the field of social neuroscience, it has become possible to investigate the neural substrates that form social hierarchy through a variety of animal models and human brain imaging methods. In perception social dominance, neural activation patterns were found to be observed in various locations of the brain, including hippocampus, amygdala, striatum, prefrontal cortex (PFC), and intraparietal sulcus.

Recruitment of glucocorticoid receptors in the hippocampus is regulated by social dominance or submission. Interestingly, the amygdala is a brain region that plays an important role during learning social rankings, social rewards, and emotional responses. The amygdala is related to the hippocampus, striatum, and PFC. However, recent studies have shown an association between social dominance and PFC. PFC is associated with stress, depressive disorder, and social dominance, and several studies have reported social behavior-related neural circuits involving PFC and amygdala in the brain of rodent, in particular, aggressive behavior and social attachment. Although the studies of neural circuits for these two behaviors have been extensively characterized, studies of social dominance behavior are still unknown.

In this regard, recent studies have shown that stress-induced changes in social dominance activate neural projections from PFC to the amygdala. In addition, phosphorylation of α-amino-3-hydroxy-5-methyl-4-isoxazolepropionic acid (AMPA)-type glutamate receptor (AMPA-R) in PFC is mediated by social dominance. In fact, the amygdala and PFC may be associated with social dominance. Phosphorylation of glutamate receptor 1 (GluA1) and glutamate receptor 2 (GluA2), which are subunits of AMPA-R, is involved in key molecules for regulation of social dominance. pS818 and pS831 of GluA1 are significantly decreased by chronic restraint stress and are required for synaptic integration during long-term potentiation (LTP). Despite some evidence for the role of AMPA-R in social dominance, the underlying molecular mechanisms involved in CREB regulation by CRTC3 are largely unknown.

Meanwhile, amphiregulin (AREG) is a protein synthesized as a type 1 transmembrane glycoprotein and has been discovered as a growth factor in various cells (Non-Patent Document 7). AREG originally inhibits growth of carcinoma cell lines; however, it is known to be a factor that promotes proliferation in normal cells such as fibroblasts, oocytes, keratinocytes, and neural stem cells. As such, AREG has bidirectional functions in proliferation, survival, motility, and angiogenesis (Non-Patent Documents 8 to 10).

Although studies on roles of AREG in the area where it is expressed are actively underway, little studies have been done on the cells that express AREG in the brain and what roles they play in the brain.

[11] [Prior Art Document]

(Non-Patent Document 1) Anacker AM, Smith ML, Ryabinin AE. Establishment of stable dominance interactions in prairie vole peers: relationships with alcohol drinking and activation of the paraventricular nucleus of the hypothalamus. Soc Neurosci. 2014;9(5):484-94.

(Non-Patent Document 2) Zhou Y, Kaiser T, Monteiro P, Zhang Sanjana N, Zhou Y, Zhang M, Zhang F, Fu Z, Feng G. Mice with Shank3 Mutations Associated with ASD and Schizophrenia Display Both Shared and Distinct Defects. Neuron. 2016 Jan 6;89(1):147-62.

(Non-Patent Document 3) Wallen-Mackenzie A, Nordenankar K, Fejgin K, Lagerstrφm MC, Emilsson L, Fredriksson R, Wass C, Andersson D, Egecioglu E, Andersson M, Strandberg J, Lindhe O, Schiφth HB, Chergui K, Hanse E, Lεngstrφm B, Fredriksson A, Svensson L, Roman E, Kullander K. Restricted cortical and amygdaloid removal of vesicular glutamate transporter 2 in preadolescent mice impacts dopaminergic activity and neuronal circuitry of higher brain function. J Neurosci. 2009 Feb 18;29(7):2238-51.

(Non-Patent Document 4) Yang CR, Bai YY, Ruan CS, Zhou HF, Liu D, Wang XF, Shen LJ, Zheng HY, Zhou XF. Enhanced aggressive behavior in a mouse model of depression. Neurotox Res. 2015 Feb;27(2):129-42.

(Non-Patent Document 5) Saxena K, Webster J, Hallas-Potts A, Mackenzie R, Spooner PA, Thomson D, Kind P, Chattarji S, Morris RGM. Experiential contributions to social dominance in a rat model of fragile-X syndrome. Proc Biol Sci. 2018 Jun 13;285(1880):20180294.

(Non-Patent Document 6) Huang WH, Wang DC, Allen WE, Klope M, Hu H, Shamloo M, Luo L. Early adolescent Rai1 reactivation reverses transcriptional and social interaction deficits in a mouse model of Smith-Magenis syndrome. Proc Natl Acad Sci US A. 2018 Oct 16;115(42): 10744-10749.

(Non-Patent Document 7) Fukuda S, Nishida-Fukuda H, Nakayama H, Inoue H, Higashiyama S. Monoubiquitination of pro-amphiregulin regulates its endocytosis and ectodomain shedding. Biochem Biophys Res Commun. 2012 Apr 6;420(2):315-20.

(Non-Patent Document 8) Shoyab M, Plowman GD, McDonald VL, Bradley JG, Todaro GJ. Structure and function of human amphiregulin: a member of the epidermal growth factor family. Science. 1989 Feb 24;243(4894 Pt 1):1074-6.

(Non-Patent Document 9) Kato M, Inazu T, Kawai Y, Masamura K, Yoshida M, Tanaka N, Miyamoto K, Miyamori I. Amphiregulin is a potent mitogen for the vascular smooth muscle cell line, A7r5. Biochem Biophys Res Commun. 2003 Feb 21;301(4): 1109-15.

(Non-Patent Document 10) Falk A, Frism J. Amphiregulin is a mitogen for adult neural stem cells. J Neurosci Res. 2002 Sep 15;69(6):757-62.

**Disclosure of Invention**

**Technical Problem**

The object of the present disclosure is to solve all of the above-mentioned problems.

An object of the present disclosure is to provide an animal model lacking social dominance and a method for producing the same.

Another object of the present disclosure is to provide a method for screening a therapeutic agent for a disease related to lack or reduction of social dominance, or a psychiatric disease, using an animal model lacking social dominance.

Yet another object of the present disclosure is to provide a composition for preventing or treating a disease related to lack or reduction of social dominance.

Still yet another object of the present disclosure is to provide a method for preventing or treating a disease related to lack or reduction of social dominance.

The object of the present disclosure is not limited to the above-mentioned objects. The objects of the present disclosure will become clearer from the following description and may be realized by means and combinations thereof as set forth in the claims.

**Solution to Problem**

Representative configurations of the present disclosure to achieve the above-metioned objects are as follows.

According to an aspect of the present disclosure, there are provided an animal model lacking social dominance, in which expression or activity of CRTC3 gene or protein is suppressed, and a method for producing the same.

According to another aspect of the present disclosure, there is provided a method for screening a therapeutic agent for a disease related to lack or reduction of social dominance, or a psychiatric disease, using the animal model lacking social dominance.

According to yet another aspect of the present disclosure, there is provided a pharmaceutical composition for preventing or treating a disease related to lack or reduction of social dominance, the composition comprising as an active ingredient an agonist for epidermal growth factor (EGF) receptor in the brain.

According to still yet another aspect of the present disclosure, a method for preventing or treating a disease related to lack or reduction of social dominance, the method comprising a step of administering an agonist to EGF receptor in the brain.

**Advantageous Effects of Invention**

The animal model lacking social dominance, in which expression or activity of CRTC3 gene or protein is suppressed, according to the present disclosure may be used as an animal model for a disease related to lack or reduction of social dominance, in particular, a disease accompanied by or caused by social defeat stress. In addition, the pharmaceutical composition comprising as an active ingredient an agonist for EGF receptor in the brain according to the present disclosure has an effect of increasing social dominance ranking of an individual, and thus can be used to effectively prevent and treat a disease related to lack or reduction of social dominance. Therefore, it is expected that the present disclosure can be used for prevention and treatment of a disease related to lack or reduction of social dominance such as chronic inflammation, self-harming, suicidal ideation, anti-social personality disorder, aggressive personality, chronic stress, anxiety neurosis, drug intoxication or mental or behavioral disorder caused by drug intoxication, schizophrenia, mood disorder, mania, depressive disorder, bipolar disorder, fragile X syndrome, autism spectrum disorder, and autism.

**Brief Description of Drawings**

FIGS. 1A to 1C illustrate results obtained by identifying expression of CRTC3 in respective regions of the brain:

FIG. 1A illustrates images obtained by performing staining of a frozen section of a WT mouse brain for CRTC3 with immunohistochemistry, in which an expression level of CRTC3 was identified in all regions of the brain, including prefrontal cortex (PFC) containing prelimbic cortex (PrL), cingulate cortex (Cg), dentate gyrus (DG), cornu ammonis (CA) 3 region, hippocampus, hypothalamus (Hy), amygdala (Amy), ventral tegmental area (VTA), and medial dorsal nucleus (MD), respectively.

FIG. 1B illustrates results obtained by performing double staining of CRTC3 and each of markers for specific cells, which are expressed in the brain, the results shown as microscopic images in which co-localization was identified using an astrocyte marker (GFAP, s100β), a neuronal marker (NeuN), and a microglial marker (Iba1), respectively.

FIG. 1C graphically illustrates results obtained by performing qRT-PCR to identify a level of CRTC3 mRNA expressed in primary astrocytes, neurons, and microglia (one-way ANOVA, ***$p < 0.001$).

FIGS. 2A to 2M illustrate results of behavioral tests which identify that social dominance is reduced in CRTR3 knockout (KO) mice (in all graphs, data are expressed as mean $\pm$ standard error of the mean (Mean $\pm$ SEM)):

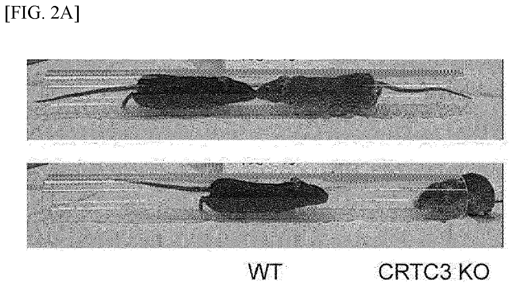

FIG. 2A illustrates a photograph related to a method of determining win/loss in a tube dominance test.

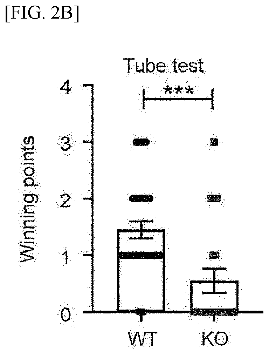

FIG. 2B illustrates winning points of WT mice and CRTC3 KO mice in a tube dominance test (WT, n = 33; CRTC3 KO, n = 20; Mann-Whitney U test, p = 0.0003).

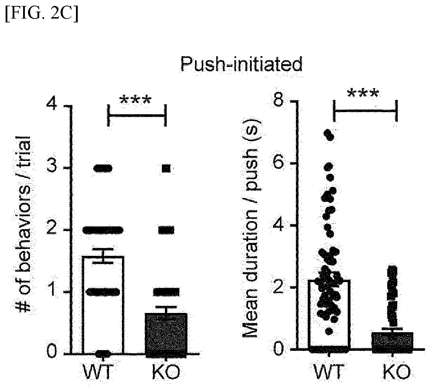

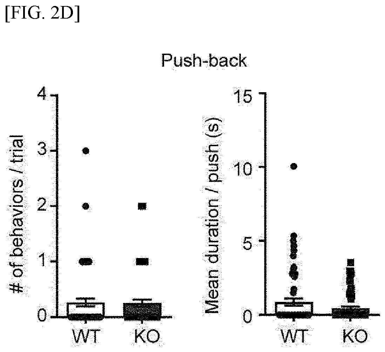

FIGS. 2C and 2D graphically illustrate number and mean duration of push-initiated (FIG. 2C) or push-back (FIG. 2D) of WT mice and CRTC3 KO mice in a tube dominance test (Mann-Whitney U test, $p < 0.0001$ for all).

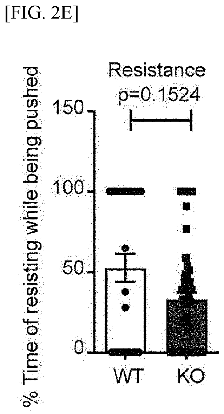

FIG. 2E graphically illustrates percentage of time of resisting while mice are pushed in a tube dominance test (Mann-Whitney U test, p = 0.1524).

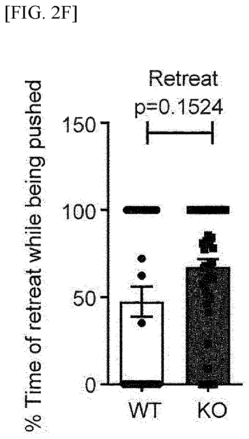

FIG. 2F graphically illustrates percentage of time of retreat while mice are pushed in a tube dominance test (Mann-Whitney U test, p = 0.1524).

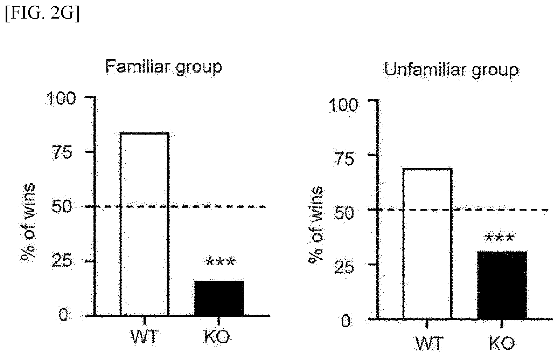

FIG. 2G illustrates results obtained by performing a tube dominance test on WT mice and CRTC3 KO mice which were raised in the same cage (left graph, Familiar group; WT, n = 51; CRTC3 KO, n = 28; tube test, n = 56; Chi-square, ***$p < 0.001$) or different cages where the mice did not encounter each other (right graph, Unfamiliar group; WT, n = 12; CRTC3 KO, n = 11; tube test, n = 55; Chi-square, *** $p < 0.001$), in which calculation was done by setting the winning mouse as 1 point and the losing mouse as 0 points.

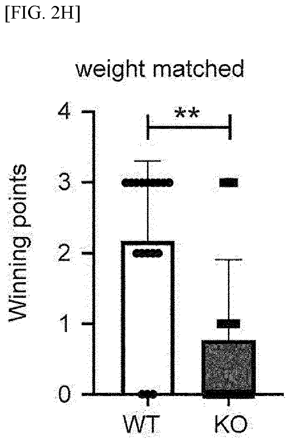

FIG. 2H illustrates results obtained by matching WT mice and CRTC3 mice of the same weight and performing a tube dominance test.

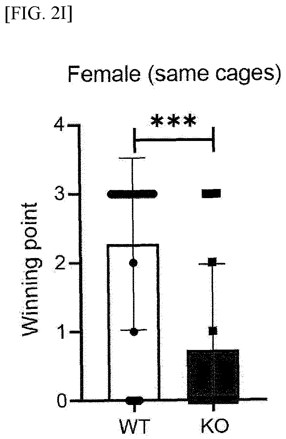

FIG. 2I illustrates results obtained by performing a tube dominance test on female WT mice and female CRTC3 KO mice which were raised in the same cage.

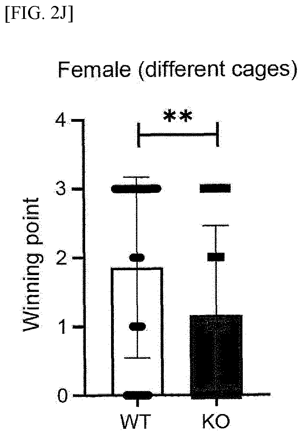

FIG. 2J illustrates results obtained by performing a tube dominance test on female WT mice and female CRTC3 KO mice which were raised in different cages.

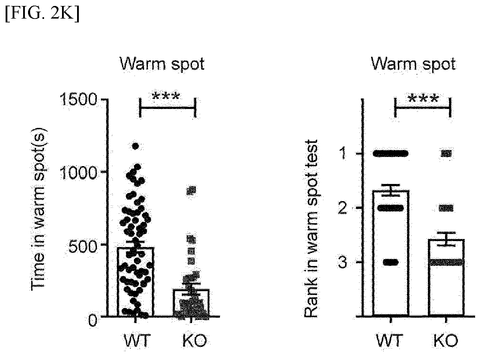

FIG. 2K graphically illustrates time (left graph) during which mice stayed in a warm spot in a warm spot test and social ranking (right graph) measured therethrough (Mann-Whitney U test, $p < 0.0001$).

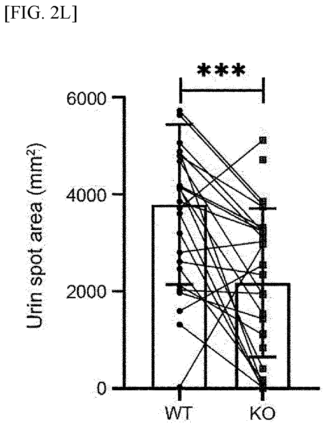

FIG. 2L graphically illustrates urine spot areas of WT mice and CRTC3 KO mice obtained by a urine marking test.

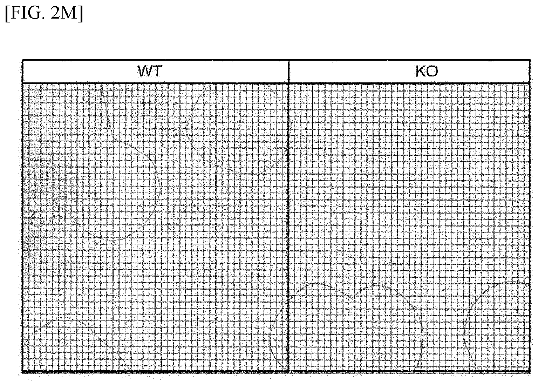

FIG. 2M illustrates a picture in which urine spots for WT mice and CRTC3 KO mice obtained by a urine marking test are respectively displayed on an OHP film with a grid pattern using ninhydrin reaction.

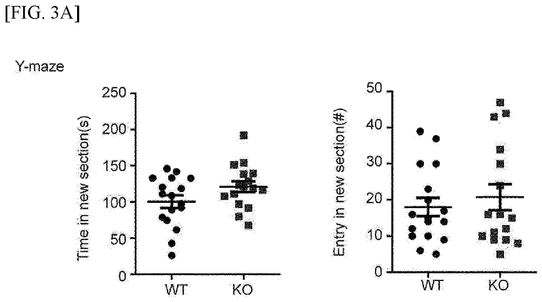

FIGS. 3A to 3D illustrate results of various behavioral tests on CRTC3 KO mice:

FIG. 3A illustrates graphs obtained by measuring, in a Y-maze test, time to explore a new arm (left graph) and number of entry (right graph) for WT mice and CRTC3 KO mice, respectively (WT, n = 17; CRTC3 KO, n = 16; t-test).

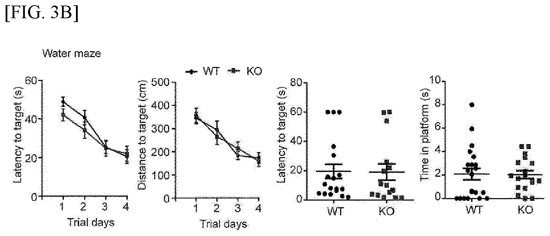

FIG. 3B illustrates results obtained by measuring, in a Morris water maze test, swimming time (s) and swimming distance (cm) to reach an escape platform, and time (s) staying in an area where the platform has been installed during a probe trial for WT mice and CRTC3 KO mice, respectively (WT, n = 21; CRTC3 KO, n = 18; t-test).

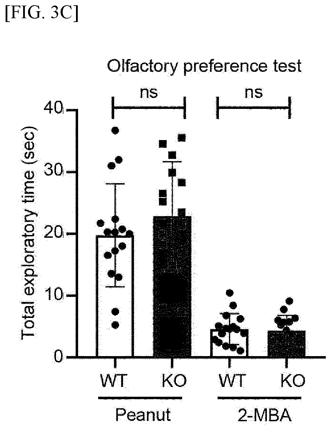

FIG. 3C illustrates results obtained by measuring, in an olfactory preference test, time to show interest for peanut butter and 2-methylbutyric acid (2-MBA) for WT mice and CRTC3 KO mice, respectively.

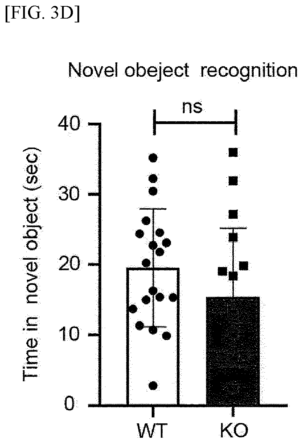

FIG. 3D illustrates results obtained by measuring, in a novel object recognition test, time to approach and explore a novel object for WT mice and CRTC3 KO mice, respectively.

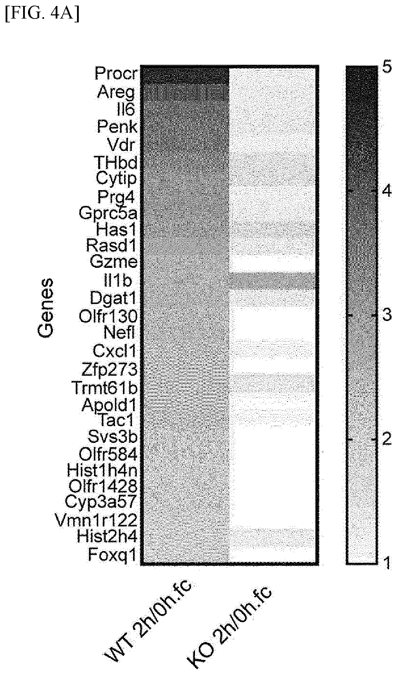

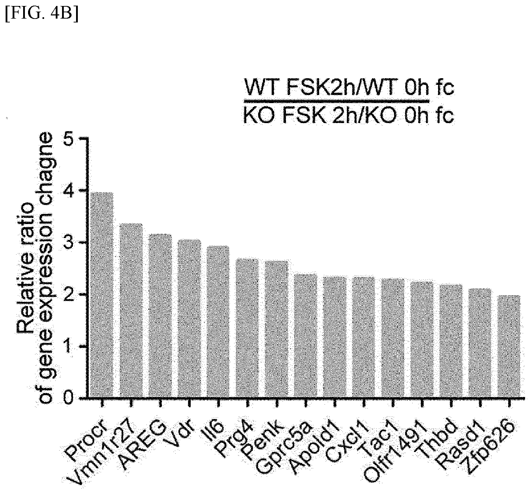

FIGS. 4A and 4B illustrate results obtained by performing whole transcript profile analysis using mouse cDNA microarray to identify expression of genes related to CRTC3:

FIG. 4A illustrates a heatmap showing expression fold-change values for genes obtained after subjecting astrocytes of WT mice and CRTC3 KO mice to treatment with 25 $\mu$M forskolin (FSK) for 2 hours.

FIG. 4B illustrates results obtained by analyzing, with microarray, relative ratio of gene expression in astrocytes of WT mice and CRTC3 KO mice, the results shown as a graph in which genes are listed in order from gene with the highest value to gene with the lowest value based on comparison of differences in transcription level for the genes in the respective mice after treatment with forskolin.

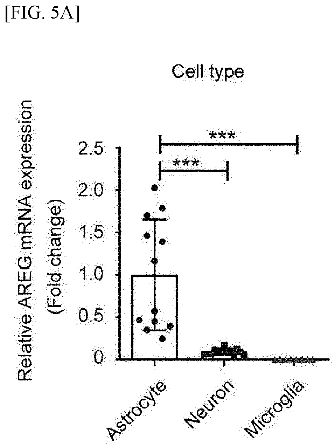

FIGS. 5A to 5F illustrate results obtained by identifying an association between AREG and CRTC3 in astrocytes, and a shape recovery effect of AREG on the cells:

FIG. 5A illustrates results obtained by synthesizing cDNA from primary astrocytes, neurons, and microglia, and performing qRT-PCR to identify cells expressing AREG (One-way ANOVA, ***$p < 0.001$).

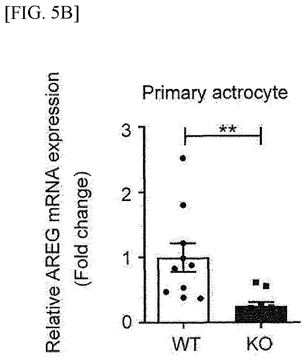

FIG. 5B illustrates results obtained by performing qRT-PCR in primary astrocyte samples from WT mice and CRTC3 KO mice and identifying expression of AREG (Unpaired t-test, **$p = 0.0041$).

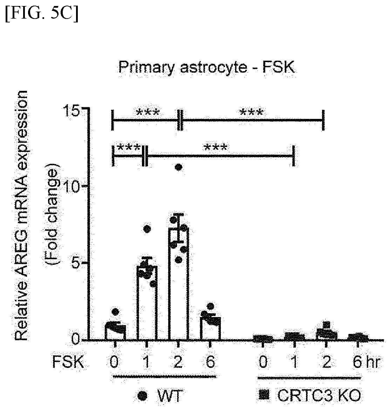

FIG. 5C illustrates results obtained by treating primary astrocytes of WT mice and CRTC3 KO mice with 25 $\mu$M forskolin (FSK) and identifying expression levels of AREG (Two-way ANOVA, *$p < 0.05$, **$p < 0.01$, ***$p < 0.001$).

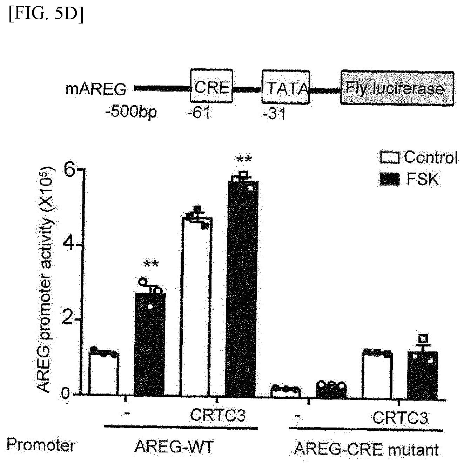

FIG. 5D illustrates results of luciferase analysis, obtained by inserting a luciferase expression gene into AREG promoter, and then measuring activity of luciferase so that an expression level of AREG is measured depending on expression of CRTC3 or treatment with forskolin (FSK).

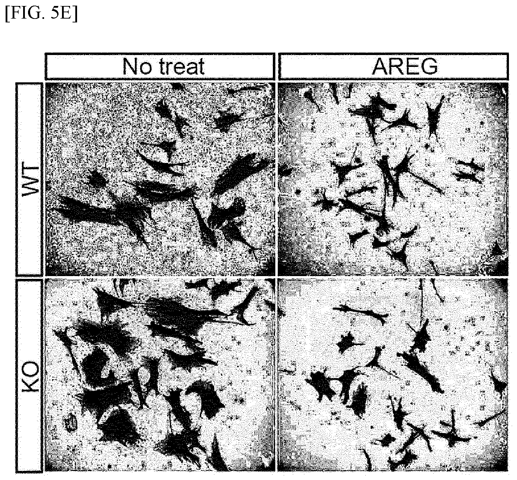

FIG. 5E illustrates images obtained by treating CRTC3 KO primary astrocytes with AREG (200 ng/ml), performing GFAP staining by immunocytochemistry, and comparing their shape with WT and AREG-untreated CRTC3 KO astrocytes.

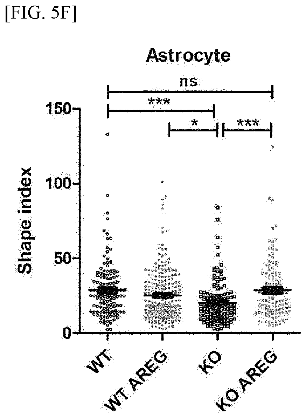

FIG. 5F illustrates results obtained by evaluating cell shape index (CIS) of CRTC3 KO astrocytes depending on treatment with AREG (One-way ANOVA, *$p < 0.05$, **$p < 0.01$).

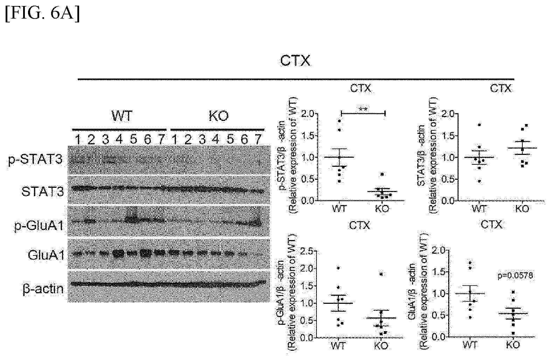

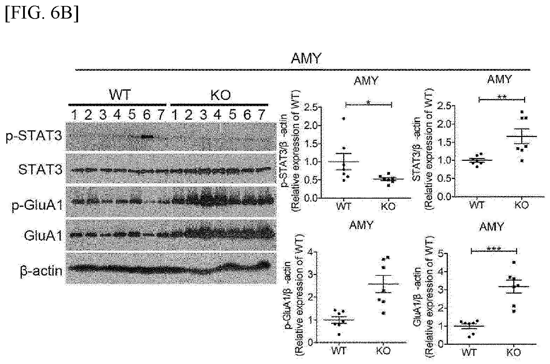

FIGS. 6A and 6B illustrate results obtained by measuring, with Western blotting, expression levels of p-STAT3, STAT3, p-GluA1, and GluA1 in cerebral cortex (CTX) and amygdala (AMY) of WT mice and CRTC3 KO mice. All bands were normalized to $\beta$-actin, and data were expressed as mean $\pm$ standard error of the mean (Mean $\pm$ SEM) in the graphs:

FIG. 6A illustrates that in the cerebral cortex (CTX) of CRTC3 KO mice, as compared with STAT3, expression of p-STAT3 is reduced (**$p = 0.0031$) and expression levels of p-GluA1 and GluA1 are slightly reduced (p-GluA1, $p = 0.0973$; GluA1, $p = 0.0578$) (Unpaired t-test, Mann-Whitney test).

FIG. 6B illustrates that in the amygdala (AMY) of CRTC3 KO mice, an expression level of p-STAT3 was significantly decreased (Mann Whitney test, *$p = 0.0379$), and the expression levels of STAT3, p-GluA1, and GluA1 were increased (STAT3 , **$p = 0.0079$; p-GluA1, **$p = 0.0021$; GluA1, ***$p = 0.0006$) (Unpaired t-test, Mann-Whitney test).

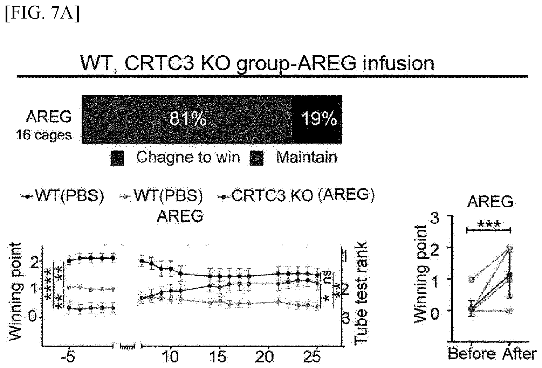

FIGS. 7A to 7F illustrate results obtained by injecting AREG, AREG-EGF, AREG-HB, or EGF into CRTC3 KO mice and low-ranked C57BL/6 mice, and measuring changes in social ranking through a tube dominance test or warm spot test. In all tube test winning-point graphs before and after drug injection, the gray graph represents a winning point for each mouse and the red graph represents changes in mean winning point:

FIG. 7A illustrates tube test results before and after AREG injection in groups of WT mice and CRTC3 KO mice.

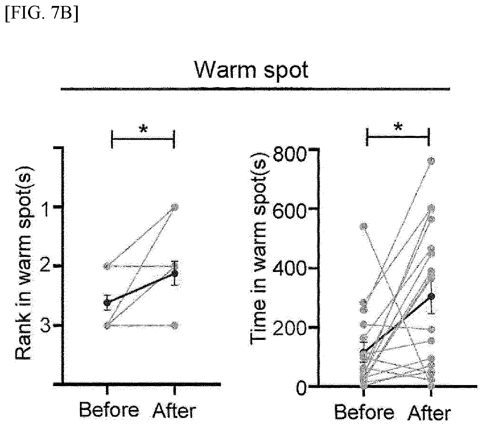

FIG. 7B illustrates changes in ranking (left graph) and time staying in a warm spot (right graph) assessed by a warm spot test before and after injection of AREG in groups of WT mice and CRTC3 KO mice (Wilcoxon signed rank test, $p = 0.0313$ (left graph), $p = 0.0129$ (right graph)).

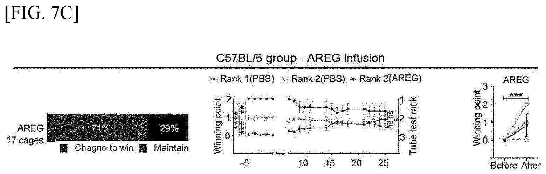

FIG. 7C illustrates results obtained by performing a tube dominance test before and after injection of AREG into the lowest-ranked mouse in a C57BL/6 mouse group.

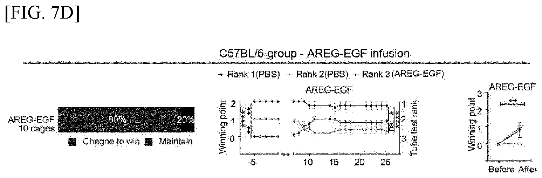

FIG. 7D illustrates results obtained by performing a tube dominance test before and after injection of AREG-EGF into the lowest-ranked mouse in a C57BL/6 mouse group.

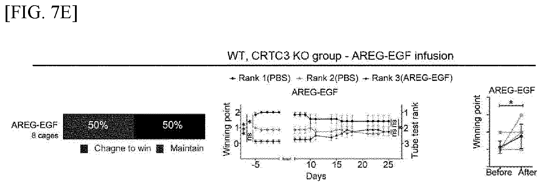

FIG. 7E illustrates results obtained by performing a tube dominance test before and after injection of AREG-EGF in groups of WT mice and CRTC3 KO mice.

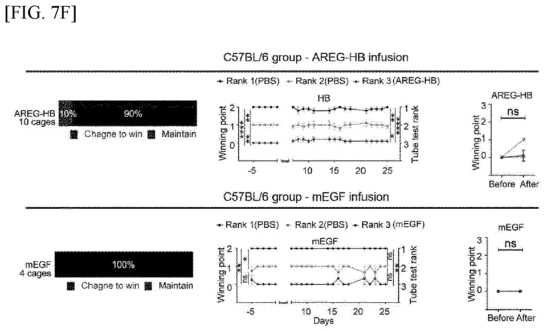

FIG. 7F illustrates results obtained by performing a tube dominance test before and after injection of AREG-HB or mouse EGF (mEGF) into the lowest-ranked mouse in a C57BL/6 mouse group.

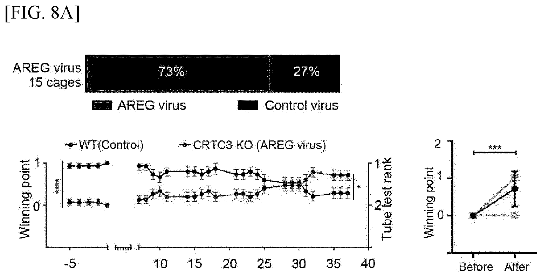

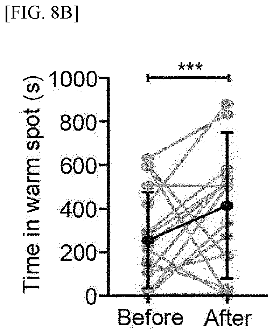

FIGS. 8A and 8B illustrate results obtained by identifying that social dominance ranking is restored in a case where amphiregulin is specifically expressed in astrocytes in CRTC3 KO mice:

FIG. 8A illustrates results of a tube dominance test performed before and after allowing AREG to be expressed specifically in astrocytes by administering AAV5-GFAP-mAREG 94-191 virus to CRTC3 KO mice.
FIG. 8B illustrates results of a warm spot test performed before and after allowing AREG to be expressed specifically in astrocytes by administering AAV5-GFAP-mAREG 94-191 virus to CRTC3 KO mice.

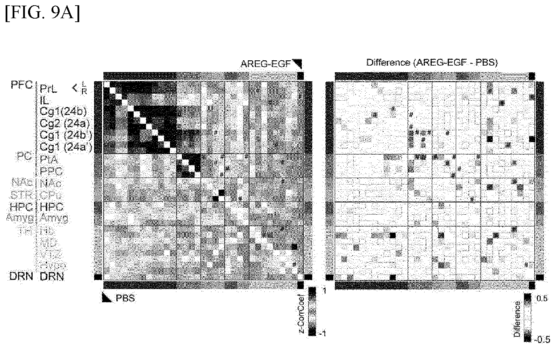

FIGS. 9A to 9B illustrate results obtained by identifying rsFC between PFC and PC depending on treatment with AREG-EGF in rats:

The left graph in FIG. 9A represents rsFC matrix of social dominance-related brain ROIs in rodents, labeled with groups of PBS (bottom left) and AREG-EGF (top right). Color bars indicate Fisher z-transformed correlation coefficient (z-CorrCoef). The right graph in FIG. 9A represents a group discrimination map expressed with the same ROIs as rsFC (AREG-EGF - PBS) (# $P < 0.05$, @ $P < 0.01$, Student's t-test). Color bars indicate differences between groups.

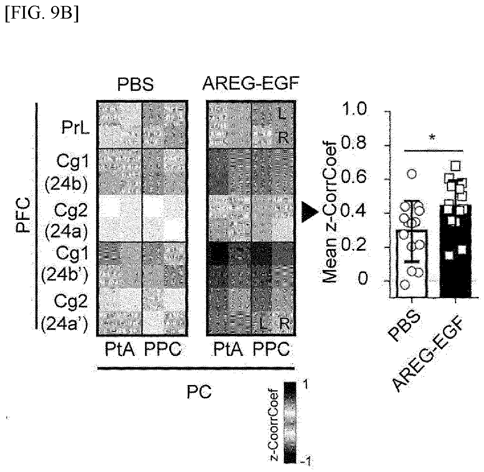

FIG. 9B illustrates mean rsFC matrix between PFC- and PC-centered ROIs (*$p < 0.05$, Student's t-test; L, left hemisphere; R, right hemisphere).

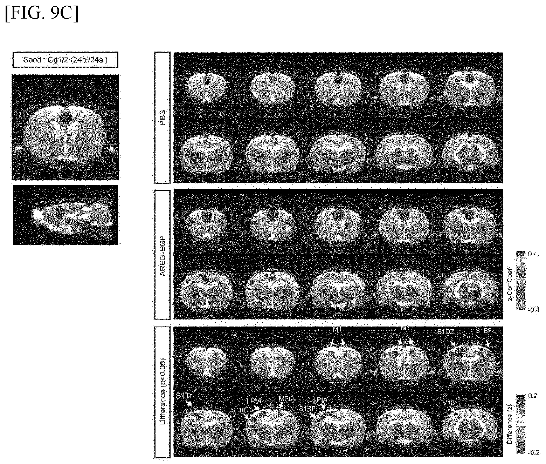

The left images in FIG. 9C represent locations of fMRI measurement, where the images were taken by setting Ca1/2 (24b'/24a') region as the center (with imaging locations at coronal section (top) and sagittal section (bottom) shown). The right images in FIG. 9c represent differences in rsFC between groups having received PBS (top) and AREG-EGF (middle) as signal changes in brain cross-section, and the bottom image shows differences between groups having received PBS and AREG-EGF.

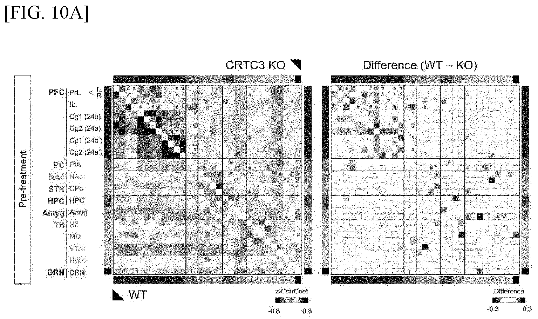

FIGS. 10A to 10E illustrate results obtained by identifying rsFC between PFC and PC depending on administration of AREG in mice:

FIG. 10A illustrates rsFC matrix (right) of social dominance-related brain ROIs in rodents before administration of AREG in WT and CRTC3 KO mice, and a group discrimination map expressed with the same ROIs as rsFC (WT - KO).

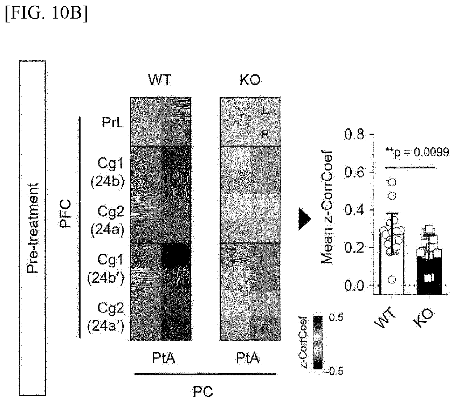

FIG. 10B illustrates mean rsFC matrix between PFC- and PC-centered ROIs before administration of AREG in WT mice and CRTC3 KO mice.

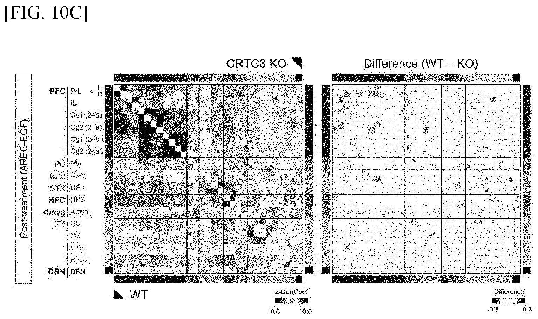

FIG. 10C illustrates rsFC matrix (right) of social dominance-related brain ROIs in rodents after administration of AREG in WT and CRTC3 KO mice, and a group discrimination map expressed with the same ROIs as rsFC (WT - KO).

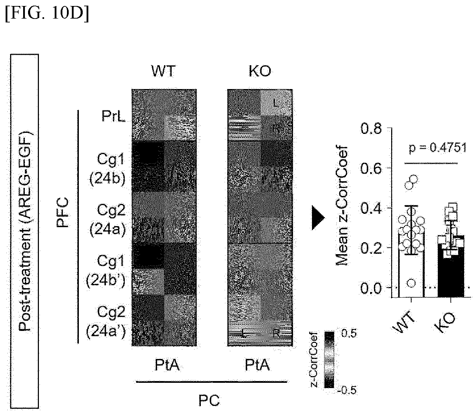

FIG. 10D illustrates mean rsFC matrix between PFC- and PC-centered ROIs after administration of AREG in WT mice and CRTC3 KO mice.

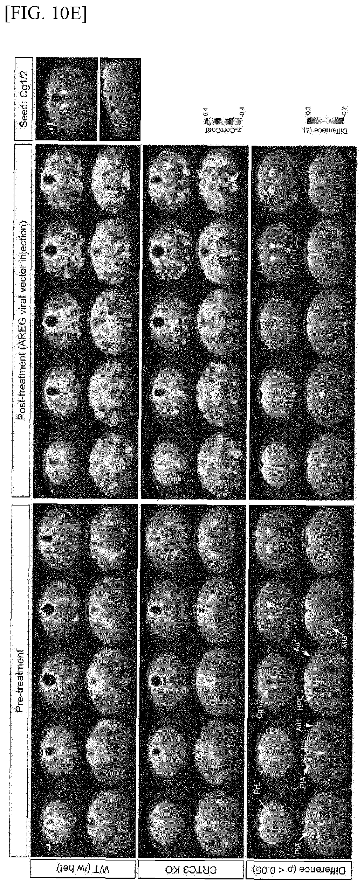

The left image in FIG. 10E represents differences in rsFC before and after administration of AREG in WT mice and CRTC3 KO mice, respectively, as signal changes in brain cross-section, and the bottom image shows differences between WT mice and CRTC3 KO mice.

The right image in FIG. 10E represents locations of fMRI measurement, where the images were taken by setting Ca1/2 region as the center.

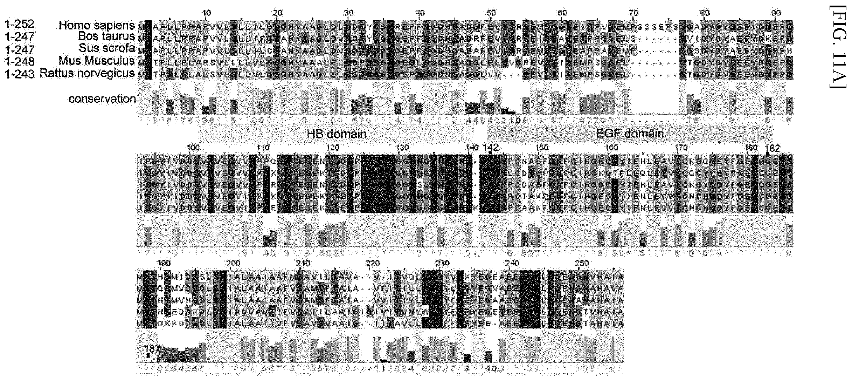

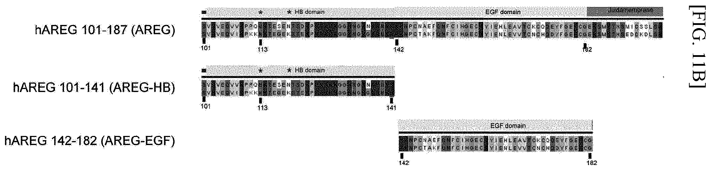

FIGS. 11A and 11B illustrate results that EGF and HB domains of AREG are conserved in various species:

FIG. 11A illustrates amino acid sequences of AREG protein in various species (Homo sapiens, human; Bos Taurus, cow; Sus scrofa, pig; Mus musculus, mouse; Rattus norvegicus, brown rat).

FIG. 11B illustrates amino acid sequences of AREG-EGF and AREG-HB domains administered to mice.

**Best Mode for Carrying out Invention**

The detailed description of the present disclosure set forth below will be described with reference to specific drawings with respect to specific embodiments in which the present disclosure may be practiced; however, the present disclosure is not limited thereto and, if properly described, is limited only by the appended claims, along with the full scope of equivalents to which such claims are entitled. It should be understood that various embodiments/examples of the present disclosure, although different, are not necessarily mutually exclusive. For example, a particular feature, structure, or characteristic described herein may be changed from one embodiment/example to another embodiment/example or implemented in combinations of embodiments/examples without departing from the technical spirit and scope of the present disclosure. Unless defined otherwise, technical and scientific terms used herein have the same meaning as generally used in the art to which the present disclosure belongs. For purposes of interpreting this specification, the following definitions will apply and whenever appropriate, terms used in the singular will also include the plural and vice versa.

**Definition**

As used herein, the term "animal model" refers to an animal that has a disease similar to a human disease or is created to be born with the disease.
The term "social dominance" is a type of social behavior that occurs among social animals living in groups or colonies and refers to all social interactions with others, including superior-subordinate relationships. The term "social dominance" may be used interchangeably with the term "social preponderance" or "social control" and may encompass the meaning of "social superiority" or "social hierarchy."
The term "social defeat stress" refers to a stress response that occurs in animals, which have become subordinate due to social defeat during social interaction or social confrontation, or animals with relatively low social dominance ranking. In the present disclosure, animals lacking or having reduced social dominance may exhibit a social defeat stress response.
The term "gene" is used broadly to refer to any nucleic acid associated with a biological function. The gene may contain a coding sequence and/or a regulatory sequence required for expression of the coding sequence.
The term "suppression of expression of a gene" refers to any action that reduces expression of a gene. Specifically, the suppression of expression of a gene may be achieved by gene knockout, gene knockdown, or introduction of a mutation such as deletion, duplication, inversion, or replacement into a gene's DNA sequence.
The term "suppression of activity of a protein" refers to any action that suppresses or inhibits activity of a target protein. The suppression of activity of a protein may mean directly inhibiting activity of a target protein or suppressing functions of a target protein by interfering with its interaction with other proteins. Substances that can suppress activity of a protein include, but are not limited to, compounds, peptides, peptide mimetics, substrate analogs, aptamers, and antibodies which specifically bind to the protein. In the present disclosure, the target protein refers to CRTC3 protein.
The term "astrocytes" is also called "star-shaped cells." The astrocytes are the most numerous in the nervous system and are known to play a role in assisting neuron activity while appropriately removing neurotransmitters secreted by neurons or regulating ion concentration in the brain.
The term "wildtype animal" is a term of the art understood by those skilled in the art and means a typical form of animal occurring in nature, as distinguished from mutant or variant forms.

The term "functional connectivity" refers to synchronized activity occurring in anatomically distinct brain regions. In other words, brain regions, which are spatially separated and exhibit temporally similar activity patterns, are considered functionally connected.

The term "agonist" refers to any compound that binds to a receptor and activates the receptor, thereby activating a biological response such as a signaling pathway. In the present disclosure, an EGF receptor agonist may refer to any compound that binds to EGF receptor and activates an EGF-induced signaling pathway.

The term "individual" is used interchangeably with "patient" and may refer to any mammal in need of prevention or treatment of lack or reduction of social dominance, such as primate (for example, human), companion animal (for example, dog and cat), domestic animal (for example, cow, pig, horse, sheep, and goat), and laboratory animal (for example, rat, mouse, and guinea pig). In an embodiment of the present disclosure, the individual is a human.

The term "treatment" generally refers to obtaining a desired pharmacological and/or physiological effect. Such an effect has a therapeutic effect in that it partially or completely cures a disease and/or harmful effects caused by the disease. Desirable therapeutic effects include, but are not limited to, prevention of occurrence or recurrence of a disease, improvement of symptoms, reduction of any direct or indirect pathological consequences of a disease, prevention of metastasis, reduction of disease progression rate, improvement or alleviation of disease state, and remission or improved prognosis. Preferably, the "treatment" may mean medical intervention for a disease or disorder that has already occurred.

The term "prevention" means obtaining a desired prophylactic pharmacological and/or physiological effect in terms of partially or completely preventing a disease or its symptoms.

The term "administration" refers to providing a substance (for example, an agonist for EGF receptor in the brain) to an individual to achieve a prophylactic or therapeutic purpose (for example, prevention or treatment of a disease related to lack or reduction of social dominance).

The full names of the abbreviations (acronyms) used herein are as shown in Table 1.

[Table 1]

| Abbreviation | Full name |
| --- | --- |
| PFC | Prefrontal cortex |
| PrL | Prelimbic cortex |
| IL | Infralimbic cortex |
| Cg1(24b) | Primary cingulate cortex anterior part |
| Cg2(24a) | Secondary cingulate cortex anterior part |
| Cg1(24b') | Primary cingulate cortex posterior part |
| Cg2(24a') | Secondary cingulate cortex posterior part |
| PC | Parietal cortex |
| PtA | Parietal associative cortex |
| PPC | Posterior parietal cortex |
| NAc | Nucleus accumbens |
| STR | Striatum |
| CPu | Caudate putamen |
| HPC | Hippocampus |
| Amyg | Amygdala |
| TH | Thalamus |
| Hb | Habenular |
| MD | Medial dorsal nucleus |
| VTA | Ventral tegmental area |
| Hypo | Hypothalamus |
| DRN | Dorsal raphe nucleus |
| M1 | Primary motor cortex |

(continued)

| Abbreviation | Full name |
| --- | --- |
| S1DZ | Primary somatosensory cortex dysgranular zone |
| S1BF | Primary somatosensory cortex barrel field |
| S1Tr | Primary somatosensory cortex trunk |
| LPtA | Lateral parietal associative cortex |
| MPtA | Medial parietal associative cortex |
| V1B | Primary visual cortex binocular area |
| L/R | Left and right bilateral ROIs |
| IH | Interhemispheric ROI |

**Animal model and method for producing same**

The present disclosure is based, in part, on the surprising discovery that CRTC3 is significantly associated with social dominance and that social dominance may become lacking or significantly reduced by inhibited function of CRTC3 in animals. According to an example of the present disclosure, it was identified that CRTC3 was expressed in various areas of the brain and CRTC3 was mainly expressed in astrocytes among various types of cells present in the brain; and to investigate functions of the CRTC3 gene, social dominance was evaluated in male and female CRTC3 KO mice, in which CRTC3 gene was knocked out, through a tube dominance test, a warm spot test, and a urine marking test. As a result, it was found that social dominance rankings (or social rankings) of the CRTC3 KO mice were significantly lower than wildtype (WT) mice (see Examples 1 and 3). In particular, the CRTC3 KO mice were found to have low social dominance ranking as compared with WT mice, both cases where the CRTC3 KO mice were raised with the WT mice and where the CRTC3 KO mice were raised separately from the WT mice. As such, in animals (in particular, social animals), deficiency or reduction in function of CRTC3 gene may cause lack or reduction of social dominance.

According to another example of the present disclosure, it was identified that the CRTC3 KO mice did not exhibit impairment of short-term or long-term memory, or changes in behaviors related to senses such as sight and smell, except for lack or reduction of social dominance (see Example 4).

As such, the present inventors have discovered that the CRTC3 gene or protein has a significant impact on behaviors related to social dominance while having little impact on other mental activities or behaviors of animals. Therefore, the animal model according to the present disclosure may be used as an animal model lacking social dominance, and may further be used as a model for a disease (for example, a psychiatric disease) related to lack or reduction of social dominance.

According to an aspect of the present disclosure, there is provided an animal model lacking social dominance in which expression or activity of CREB-regulated transcription coactivator 3 (CRTC3) gene or protein is suppressed. In addition, there is provided a method for producing an animal model lacking social dominance, comprising a step of suppressing expression or activity of CRTC3 gene or protein.

In an embodiment, the animal model may be such that expression or activity of CRTC3 gene or protein is specifically suppressed in the brain. In addition, the animal model may be such that expression or activity of CRTC3 gene or protein is specifically suppressed in astrocytes among cells existing in the brain.

In another embodiment, the producing method may comprise a step of suppressing expression or activity of CRTC3 gene or protein specifically in the brain of an animal. In addition, the producing method may comprise a step of suppressing expression or activity of CRTC3 gene or protein specifically in brain astrocytes in an animal.

The "suppressed expression or activity of CRTC3 gene or protein" means that functions of CRTC3 gene or protein are inhibited or hindered. Such inhibition or hindrance can be achieved by interfering with transcription of CRTC3 gene or inhibiting translation of the mRNA of CRTC3 gene. Alternatively, activity of CRTC3 protein can be reduced or inactivated by specific binding to an active site of the CRTC3 protein or induction of structural modification thereof.

In another embodiment, the animal model may be produced by knocking out or knocking down CRTC3 gene. As described above, suppressed expression of CRTC3 gene may be achieved by introducing a mutation such as deletion, duplication, inversion, or replacement into the gene's DNA sequence, in addition to knocking out or knocking down the gene.

Here, the term "knock out" means introducing DNA, which causes a gene defect, from outside to induce complete inactivation of a normal gene, and the term "knock down" means degrading expression of mRNA of a gene using, for example, RNAi, to decrease an expression level of the gene. For example, CRTC3 knockout animals have their CRTC3 gene inactivated, destroyed, deleted, or "knocked out."

In another embodiment, the animal model may be such that activity of CRTC3 protein is suppressed. As described above, suppression of activity of CRTC3 protein may be achieved by compounds, peptides, peptide mimetics, substrate analogs, aptamers, antibodies, and the like which specifically bind to the CRTC3 protein, and known techniques may be used without limitation for this purpose.

In yet another embodiment, the animal model may be characterized by reduced expression or activity of amphiregulin (AREG) gene or protein as compared with a wild type (WT) animal. As described below, CRTC3 directly regulates expression of AREG, which is a target gene of CREB, and thus the animal model is such that expression or activity of AREG is significantly reduced, especially in the brain. It is believed that impairment of social dominance is caused by lack or reduction of AREG.

In addition, the animal model may not exhibit changes in memory-related behavior, sensory-related behavior, or both, as compared with a WT animal. Due to these characteristics, the animal model of the present disclosure may be used as a model for various diseases or conditions related to lack or reduction of social dominance.

In addition, the animal model may have reduced functional connectivity between prefrontal cortex (PFC) and parietal cortex (PC) in the brain, as compared with a wild-type animal. According to an example, it was identified that CRTC3 KO mice had significantly lower rsFC (resting state functional connectivity) between PFC and PC as compared with WT mice, which shows that CRTC3 plays an important function in connectivity between PFC and PC regions in the brain (see Example 11).

In yet another embodiment, the animal model may be used as an animal model for a disease related to lack or reduction of social dominance.

The disease may be accompanied by or caused by social defeat stress, and the details are the same as those described in the section "Pharmaceutical composition and method for preventing or treating disease using same" below.

In an embodiment, the disease accompanied by or caused by social defeat stress may be at least one selected from the group consisting of chronic inflammation, self-harming, suicidal ideation, anti-social personality disorder, aggressive personality, chronic stress, anxiety neurosis, drug intoxication or mental or behavioral disorder caused by drug intoxication, schizophrenia, mood disorder, mania, depressive disorder, bipolar disorder, fragile X syndrome, autism spectrum disorder, and autism. The details related to each disease are the same as those described in the section "Pharmaceutical composition and method for preventing or treating diseases using same" below.

Animals, which can be used in the above animal model, are animals that have various diseases occurring in humans. The animals include, without limitation, animals other than humans which may serve as an animal model used for investigation of human diseases and development of treatments, such as long-term genetic research that requires several generations or organ transplant experiments that are difficult to conduct directly on humans. Specifically, the animal may be selected from the group consisting of mice, rats, guinea pigs, monkeys, dogs, cats, rabbits, cows, sheep, pigs, and goats. Among the above animals, mice or rats are advantageous in that they have good reproductive ability, developmental ability, and ability to adapt to the environment, have a large number of offspring, thereby allowing a large number of individuals to be obtained in an experimental group so that reliable results are obtained, and are easy to handle. Thus, in specific examples of the present disclosure, the mice and rats were used, but the animals are not limited thereto.

In an embodiment, suppression of CRTC3 gene or protein for production of an animal model lacking social dominance may be achieved by a known method based on known information about CRTC3 gene or protein.

Table 2 provides exemplary information about CRTC3 gene (ORF of CRTC3 mRNA) and protein for each of mouse (*Mus musculus*), rat (*Rattus norvegicus*), and monkey (*Macaca mulatto*). In another embodiment, in a case of producing an animal model of the present disclosure using mice, it is possible for a person skilled in the art to knock out or knock down CRTC3 DNA (or mRNA) having the nucleotide sequence of SEQ ID NO: 1. Alternatively, it is possible to inhibit activity of CRTC3 using a substance specific to CRTC3 protein having the amino acid sequence of SEQ ID NO: 2.

The same applies to cases where rats, monkeys, or the like are used as model animals. Meanwhile, the CRTC3 sequences for specific animals shown in Table 2 are exemplary, and DNA or protein sequences of CRTC3 for other animals can be easily found in the database provided by the U.S. National Center for Biological Information (www.ncbi.nlm.nih.gov).

[Table 2]

| Item | Type | Sequence | Sequence listing |
|---|---|---|---|
| Mouse CRTC3 | DNA | ATGGCCGCCTCGCCCGGTTCGGGCAGCGCCAACCCGCGGAAGTTCAG<br>TGAGAAGATCGCCCTGCACACGCAGCGGCAGGCCGAGGAGACGCGGG<br>CCTTCGAGCAGCTCATGACCGACCTCACCCTGTCCCGGGTTCAGTTT<br>CAGAAGCTTCAGCAGCTGCGCCTTACCCAGTACCACGGGGGATCCTT<br>ACCCAACGTGAGCCAGCTGCGGAACAGCGCGCCAGAGTTCCAGCCAT<br>CACTTCATCAAGCCGATAATGTTCGTGGAACCCGCCACCATGGGCTG<br>GTGGAGAGGCCAGCCCGGAACCGCTTCCACCCCCTTCACCGAAGGTC<br>TGGGGACAAGCCAGGGAGACAGTTTGATGGGAATGCTTTCGCAGCCA<br>GTTATTCTTCACAGCACCTGGATGAGAGCTGGCCACGGCAACAGCCT<br>CCTTGGAAAGAAGAGAAGCATCCTGGATTCAGGCTAACATCTGCACT<br>TAACAGGACCAATTCTGATTCTGCTCTTCACACGAGTGCTCTGAGCA<br>CCAAACCCCAGGACCCCTATGGAGGAGGAGGCCAGTCAGCCTGGCCT<br>GCCCCATACATGGGTTTCTGTGATGGTGAAAACGATGGCCATGCGGA<br>AGTAGCCGCCTTCCCGGGTCCGCTGAAAGAAGAGAATCTGTTAAATG<br>TCCCGAAGCCACTGCCTAAACACCTGTGGGAGAGCAAGGAGATCCAG<br>TCCCTGTCTGGGCGCCCTCGATCCTGTGATGTTGGGGGCGGCAATGC<br>TTTCCCACATAATGGCCAGAACACAGGCCTCTCACCGTTCCTGGGCA<br>CCCTGAACACCGGAGGGTCGTTACCAGATCTAACCAACCTCCACTAC<br>TCAGCACCCCTGCCAGCCTCCCTGGACACCAGCGACCACCTCTTTGG<br>TAGCATGAGTGTGGGGAACAGCGTGGGCAACCTTCCAGCTGCCATGA<br>CTCACCTGGGGATAAGAACCTCCTCTGGTCTCCAAAGTTCTCGAAGT<br>AACCCTTCCATCCAAGCCACACTCAGTAAGATGGCACTCTCCTCGTC<br>CCTCAAGTGCCACCCGCAGCCGTCTGTGGCCAACGCCTCTGCTCTGC<br>ACCCCTCCCTCCGGCTCTTCTCCCTTAGCAACCCGTCTCTTTCCACC<br>ACAAACCTGAGTGGCCCCTCTCGGCGCAGGCAGCCTCCAGTCAGCCC<br>TCTCACGCTCTCTCCTGGCCCTGAAGCACATCAAGGCTTCAGCAGAC<br>AGCTCTCTGCGACCAGCCCACTGAACCCGTATCCTGCCTCCCAGATG<br>GTGACCTCAGAGCAGAGCCCACTTTCCTTCCTGCCCACAGACGCTCA<br>*(continued on next page)* | SEQ ID NO: 1 |

(continued)

| Item | Type | Sequence | Sequence listing |
|------|------|----------|------------------|
| Mouse CRTC3 | DNA | (*continuing from previous page*)<br>AGCCCAAGTGTCCCCACCACCCCCTTACCCCACACCCCAGGAGCTGC<br>CTCAGCCACTCCTACAGCAGCCCCATGCCCAGGAGCCCCCAACTCAG<br>CAGCCCCAGGCAGCCCCCTCCCTGCCACAGTCAGACTTCCAGCTCCT<br>CACTGCCCAGGGCTCAGCTTTGACCAGCTTCTTCCCGGATGTGCGCT<br>TTGACCAACAGCCCATGAGGCCCAGCCCTGCCTTTCCTCAGCAGGTG<br>CCTTTGGTACAGCAGAGCCACCGAGAACCACAGGACTCATTCCACTT<br>GAGACCAAACCCATATTCCAGCTGTGGCAGCTTCCCAGGCACCATCC<br>TGACAGAAGATACAAACAGCAACCTGTTCAAAGGCCTTAGCGGGGGG<br>CTGTCGGGCATGCCAGAGGTCAGCCTGGACATGGACACCCCGTTCCC<br>TCTGGAAGAGGAGCTGCAGATCGAACCCTTGAGCCTCGATGGACTCA<br>ACATGCTAAGTGACTCCAGCATGGGCCTGCTGGACCCCTCTGTGGAG<br>GAGACGTTTCGAGCTGACCGGCTATGA | SEQ ID NO: 1 |
| Mouse CRTC3 | Protein | MAASPGSGSANPRKFSEKIALHTQRQAEETRAFEQLMTDLTLSRVQF<br>QKLQQLRLTQYHGGSLPNVSQLRNSAPEFQPSLHQADNVRGTRHHGL<br>VERPARNRFHPLHRRSGDKPGRQFDGNAFAASYSSQHLDESWPRQQP<br>PWKEEKHPGFRLTSALNRTNSDSALHTSALSTKPQDPYGGGGQSAWP<br>APYMGFCDGENDGHAEVAAFPGPLKEENLLNVPKPLPKHLWESKEIQ<br>SLSGRPRSCDVGGGNAFPHNGQNTGLSPFLGTLNTGGSLPDLTNLHY<br>SAPLPASLDTSDHLFGSMSVGNSVGNLPAAMTHLGIRTSSGLQSSRS<br>NPSIQATLSKMALSSSLKCHPQPSVANASALHPSLRLFSLSNPSLST<br>TNLSGPSRRRQPPVSPLTLSPGPEAHQGFSRQLSATSPLNPYPASQM<br>VTSEQSPLSFLPTDAQAQVSPPPPYPTPQELPQPLLQQPHAQEPPTQ<br>QPQAAPSLPQSDFQLLTAQGSALTSFFPDVRFDQQPMRPSPAFPQQV<br>PLVQQSHREPQDSFHLRPNPYSSCGSFPGTILTEDTNSNLFKGLSGG<br>LSGMPEVSLDMDTPFPLEEELQIEPLSLDGLNMLSDSSMGLLDPSVE<br>ETFRADRL | SEQ ID NO: 2 |

(continued)

| Item | Type | Sequence | Sequence listing |
|------|------|----------|------------------|
| Rat CRTC3 | DNA | ATGGCCGCCTCGCCCGGCTCGGGCAGCGCCAACCCGCGGAAGTTCAG TGAGAAGATCGCGCTGCACACGCAGCGGCAGGCCGAGGAGACGCGGG CCTTCGAGCAGCTCATGACCGACCTCACCCTGTCCCGGGTTCAGTTT CAGAGGCTTCAGCAACTGCGCCTTACACAGCACCACGGGGGGATCCTT ACCTAATGTGAGCCAGCTGCGGGGCAGCGCACCAGAGCTTCAGCCGT CACTCCATCACGCTGATAATGTCCGTGGGACCCGCCACCATGGGCTG GTGGAGAGGCCAGCCCGGAACCGCTTCCACCCTCTTCACCGCAGGTC TGGGGACAAGCCAGGGAGACAGTTTGATGGAAATGCTTTTGCAGCCA GTTATTCTTCGCAGCCCCTGGATGAGAGCTGGCCAAGGCAACAGCCT CCATGGAAAGAGGAAAAGCATCCTGGATTCAGGCTGACATCTGCACT TAACAGGACCAATTCTGATTCTGCTCTTCACACGAGTGCTCTGAGCA CCAAACCCCAGGACCCCTATGGAGGAGGAGGCCAGTCAGCCTGGCCT GCCCCATACATGGGTTTCTGTGATGGTGAAAATGATGGCCGTGGGGA AGCCGCCTTCCCCGGTCTGCTGAAGGAAGAGAATCTGTTAAACGTCC CGAAGCCACTGCCCAAACAACTGTGGGAGACCAAGGAGATCCAGTCC CTGTCTGGACGCCCTCGGTCCTGTGATGTTGGGGGCGGCAGTGCCTT CCCACATAATGGCCAAAACACAGGCCTCTCGCCATTTCTGGGGACCC TGAACACTGGAGGGTCATTACCAGATCTAACCAACCTTCACTACTCA GCACCCCTGCCAGCCTCCCTGGACACCAGTGACCACCTCTTTGGTAG CATGAGCGTGGGGAACAGTGTGGGCAACCTTCCAGCTGCTATGACTC ACCTGGGGATAAGAAACTCCTCTGGTCTCCAGAGTTCTCGAAGTAAC CCTTCCATCCAAGCCACGCTCAATAAGATTGCGCTGTCCTCATCCCT CAATAGCCACCCACAGCCGTCCGTGGCCAATGCCTCTGCTCTCCACC CCTCGCTCCGTCTCTTCTCCCTTAGCAACCCATCTCTTTCTACCACA AACCTGAGTGGCCCCTCTCGGCGCAGGCAGCCTCCAGTCAGCCCTCT CACGCTCTCTCCTGGCCCTGAAGCACATCAAGGTTTCAACAGACAGC TATCTGCAACCAGCCCACTGAACCCGTACCCTGCCTCCCAGATGGTG ACCTCAGAGCAGAGTCCACTTTCCTTCCTGCCCACAGACGCTCAAGC | SEQ ID NO: 3 |

*(continued on next page)*

(continued)

| Item | Type | Sequence | Sequence listing |
|------|------|----------|------------------|
| Rat CRTC3 | DNA | (*continuing from previous page*)<br>CCAAGTGTCCCCACCACCCCCTTACCCCACACCCCAGGAGCTGCCTC<br>AACCACTCCTACAGCAGCCCCATGCCCATGAGCCCCCAGCTCAGCAG<br>CCCCAGGCAGCCCCCTCCCTGTCACAGTCAGACTTCCAGCTCCTCAC<br>TGCCCAGGGCTCGTCTCTGACCAACTTCTTCCGCGATGTGGGCTTTG<br>ACCAGCAGCCCATGAGGCCCAGCCCCGCCTTTCCTCAGCAGGTGCCT<br>TTGGTACAGCAGAGCCACCGAGAACCACAGGACTCGTTCCACTTGAG<br>ACCAAACCCATATTCCAACTGTGGGAGCTTCCCGAACACCATCCTGA<br>CAGAAGATACCAACACCAGCCTGTTCAAAGGCCTTAGTGGGGGGCTG<br>TCGGGCATGCCTGAGGTCAGCCTGGACATGGACACTCCGTTCCCGCT<br>GGAAGAGGAGCTACAGATCGAACCCTTGAGCCTGGACGGACTCAACA<br>TGTTAAGTGACTCCAGCATGGGCCTGCTGGACCCCTCTGTGGAGGAG<br>ACATTTCGAGCTGACCGGCTGTGA | SEQ ID NO: 3 |
| Rat CRTC3 | Protein | MAASPGSGSANPRKFSEKIALHTQRQAEETRAFEQLMTDLTLSRVQF<br>QRLQQLRLTQHHGGSLPNVSQLRGSAPELQPSLHHADNVRGTRHHGL<br>VERPARNRFHPLHRRSGDKPGRQFDGNAFAASYSSQPLDESWPRQQP<br>PWKEEKHPGFRLTSALNRTNSDSALHTSALSTKPQDPYGGGGQSAWP<br>APYMGFCDGENDGRGEAAFPGLLKEENLLNVPKPLPKQLWETKEIQS<br>LSGRPRSCDVGGGSAFPHNGQNTGLSPFLGTLNTGGSLPDLTNLHYS<br>APLPASLDTSDHLFGSMSVGNSVGNLPAAMTHLGIRNSSGLQSSRSN<br>PSIQATLNKIALSSSLNSHPQPSVANASALHPSLRLFSLSNPSLSTT<br>NLSGPSRRRQPPVSPLTLSPGPEAHQGFNRQLSATSPLNPYPASQMV<br>TSEQSPLSFLPTDAQAQVSPPPPYPTPQELPQPLLQQPHAHEPPAQQ<br>PQAAPSLSQSDFQLLTAQGSSLTNFFRDVGFDQQPMRPSPAFPQQVP<br>LVQQSHREPQDSFHLRPNPYSNCGSFPNTILTEDTNTSLFKGLSGGL<br>SGMPEVSLDMDTPFPLEEELQIEPLSLDGLNMLSDSSMGLLDPSVEE<br>TFRADRL | SEQ ID NO: 4 |

(continued)

| Item | Type | Sequence | Sequence listing |
|---|---|---|---|
| Monkey CRTC3 | DNA | ATGGCCGCCTCGCCGGGCTCGGGCAGCGCCAACCCGCGGAAGTTCAG TGAGAAGATCGCGCTGCACACGCAGCGACAGGCCGAGGAGACGCGGG CCTTCGAGCAGCTCATGACCGACCTCACCCTGTCGCGGGTTCAATTT CAGAAGCTTCAGCAACTGCGCCTTACACAGTACCATGGAGGATCCTT ACCAAATGTGAGCCAGCTGCGGAGCAGTGCGTCAGAGTTTCAGCCGT CATTTCACCAAGCTGATAATGTTCGGGGAACCCGCCATCACGGGCTG GTGGAGAGGCCATCCAGGAACCGCTTCCACCCCCTGCACCGAAGGTC TGGGGACAAGCCAGGGCGGCAATTTGATGGTAGTGCTTTTGGAGCCA ATTATTCCTCACAGCCTCTGGATGAGAGTTGGCCAAGGCAGCAGCCT CCTTGGAAAGACGAAAAGCATCCTGGGTTCAGGCTGACATCTGCACT TAACAGGACCAATTCTGATTCTGCTCTTCACACGAGTGCTCTGAGCA CCAAGCCCCAGGACCCCTATGGAGGAGGGGGGCCAGTCGGCCTGGCCT GCCCCATACATGGGTTTCTGTGATGGTGAGAATAATGGACATGGGGA AGTAGCATCTTTCCCTGGCCCATTGAAAGAAGAGAATCTGTTAAATG TTCCGAAGCCAGTGCCAAAACAACTGTGGGAGACCAAGGAGATTCAA TCCCTGTCAGGACGCCCTCGATCCTGTGATGTTGGAGGTGGCAATGC TTTTCCACATAATGGTCAAAACCTAGGCCTTTCACCCTTTTTGGGGA CCCTGAATACTGGAGGGTCATTGCCAGATCTAACCAACCTCCACTAC TCCACGCCCCTGCCAGCCTCCCTGAACACCACCGACCACCTCTTCGG GAGTATGAGTGTGGGGAATAGTGTGAACAACATCCCAGCTGCTGTGA CCCACCTGGGTATAAGAAGCTCCTCTGGTCTCCAGAGTTCTCGGAGT AACCCCTCCATCCAAGCCACGCTCAATAAGACTGTGCTTTCCTCTTC CCTAAATAACCACCCGCAGACATCTGTTCCCAATGCATCTGCTCTTC ACCCTTCACTCCGTCTATTTTCCCTTAGCAACCCATCTCTTTCCACC ACAAACCTGAGTGGCCCATCTCGGCGTCGGCAGCCTCCCGTCAGTCC TCTCACCCTTTCTCCTGGCCCCGAAGCACATCAAGGTTTCAGCAGAC AGCTGTCTTCAACCAGCCCACTGGCCCCATATCCTACCTCCCAGATG GTGTCCTCAGACCGAAGCCAACTTTCCTTCCTGCCCACAGAAGCTCA AGCCCAGGTGTCCCCGCCACCCCCTTACCCTGCGCCCCAGGAGCTCA | SEQ ID NO: 5 |

(continued)

| Item | Type | Sequence | Sequence listing |
|---|---|---|---|
| Monkey CRTC3 | DNA | (continuing from previous page)<br>CCCAGCCCCTCCTGCAGCAGCCCCGCACCCCTGAGGCCCCTGCCCAG<br>CAGCCCCAGGCAGCCTCCTCACTGCCACAGTCGGACTTTCAGCTTCT<br>CCCAGCCCAGGGCTCATCTTTGACCAACTTCTTCCCAGATGTGGGTT<br>TTGACCAGCAGTCCATGAGGCCAGGCCCTGCCTTTCCTCAACAGGTG<br>CCTCTAGTGCAACAAGGTTCCCGAGAACTGCAGGACTCTTTTCATTT<br>GAGACCAAGCCCATATTCCAACTGCGGGAGTTTCCCAAACACCATCC<br>TGACAGAAGACTCCAGCACCAGCCTGTTCAAAGACCTCAACAGTGCG<br>CTCGCAGGCCTGCCCGAGGTCAGCCTGAACGTGGACACTCCATTTCC<br>ACTGGAAGAGGAGCTGCAGATTGAACCCCTGAGCCTGGACGGACTCA<br>ACATGTTAAGTGACTCCAGCATGGGCCTGCTGGACCCCTCTGTTGAA<br>GAGACGTTTCGAGCTGACAGACTGTGA | SEQ ID NO: 5 |
| Monkey CRTC3 | Protein | MAASPGSGSANPRKFSEKIALHTQRQAEETRAFEQLMTDLTLSRVQF<br>QKLQQLRLTQYHGGSLPNVSQLRSSASEFQPSFHQADNVRGTRHHGL<br>VERPSRNRFHPLHRRSGDKPGRQFDGSAFGANYSSQPLDESWPRQQP<br>PWKDEKHPGFRLTSALNRTNSDSALHTSALSTKPQDPYGGGGQSAWP<br>APYMGFCDGENNGHGEVASFPGPLKEENLLNVPKPVPKQLWETKEIQ<br>SLSGRPRSCDVGGGNAFPHNGQNLGLSPFLGTLNTGGSLPDLTNLHY<br>STPLPASLNTTDHLFGSMSVGNSVNNIPAAVTHLGIRSSSGLQSSRS<br>NPSIQATLNKTVLSSSLNNHPQTSVPNASALHPSLRLFSLSNPSLST<br>TNLSGPSRRRQPPVSPLTLSPGPEAHQGFSRQLSSTSPLAPYPTSQM<br>VSSDRSQLSFLPTEAQAQVSPPPPYPAPQELTQPLLQQPRTPEAPAQ<br>QPQAASSLPQSDFQLLPAQGSSLTNFFPDVGFDQQSMRPGPAFPQQV<br>PLVQQGSRELQDSFHLRPSPYSNCGSFPNTILTEDSSTSLFKDLNSA<br>LAGLPEVSLNVDTPFPLEEELQIEPLSLDGLNMLSDSSMGLLDPSVE<br>ETFRADRL | SEQ ID NO: 6 |

## Method for screening therapeutic agent

According to another aspect of the present disclosure, there is provided a method for screening a therapeutic agent for a disease related to lack or reduction of social dominance or a psychiatric disease using an animal model lacking social dominance. In an embodiment, the screening method may comprise steps of (a) administering a candidate drug to the animal model lacking social dominance and (b) determining whether social dominance of the animal model is improved.

In another embodiment, the step of determining whether social dominance of the animal model is improved may comprise step (b-1) of measuring social dominance rankings in the animal model before and after treatment with the candidate drug and comparing the rankings with each other. Here, the social dominance ranking may be measured, evaluated, and compared by known behavioral test methods, or the tube dominance test, warm spot test, urine marking test, or the like as described in detail herein.

In a case where the present screening method comprises the step (b-1), the screening method may further comprise step (c-1) of selecting the candidate drug as a therapeutic agent for a disease related to lack or reduction of social dominance when the social dominance ranking increases after treatment with the candidate drug as compared with before treatment with the candidate drug in the animal model.

In yet another embodiment, the step of determining whether social dominance of the animal model is improved may comprise step (b-2) of measuring expression or activity levels of amphiregulin before and after treatment with the candidate drug in the animal model or a biological sample therefrom and comparing the levels with each other. Here, the measurement of the expression or activity levels of amphiregulin may be performed by measuring a transcription level of mRNA of amphiregulin or an expression or activity level of amphiregulin protein. The measurement of the transcription level of mRNA may be performed by one or more methods of measuring a transcription level of mRNA selected from the group consisting of polymerase chain reaction (PCR), reverse transcription polymerase chain reaction (RT-PCR), real-time polymerase chain reaction (real-time PCR), RNase protection assay (RPA), microarray, and northern blotting, but the method is not limited thereto. The measurement of the expression or activity level of amphiregulin protein may be performed by one or more methods of measuring an expression or activity level of protein selected from the group consisting of Western blotting, immunostaining, radioimmunoassay (RIA), radioimmunodiffusion, enzyme-linked immunosorbent assay (ELISA), immunoprecipitation, flow cytometry, immunofluorescence, Ouchterlony immunodiffusion, Rocket immunoelectrophoresis, complement fixation assay and protein chip, but the method is not limited thereto.

In a case where the present screening method comprises the step (b-2), the screening method may further comprise step (c-2) of selecting the candidate drug as a therapeutic agent for a disease related to lack or reduction of social dominance when the expression or activity level of amphiregulin increases after treatment with the candidate drug as compared with before treatment with the candidate drug in the animal model or a biological sample therefrom.

In still yet another embodiment, the determination step (step (b)) may be performed through electroencephalogram (EEG) analysis or brain MRI analysis, in addition to behavioral tests for evaluating social dominance and measurement of an expression or activity level of amphiregulin, or any combination thereof.

## Pharmaceutical composition and method for preventing or treating disease using same

The present disclosure is also based on the surprising discovery that social dominance ranking (or social ranking) may be changed in a case where a substance (for example, amphiregulin or a fragment thereof) capable of activating EGF receptor is administered to an animal lacking or having reduced social dominance. The present inventors have discovered an association between CRTC3 and amphiregulin (AREG) and identified, through experiments, that AREG, in particular, the EGF domain of AREG, plays a key role in restoring impaired social dominance in animals. Therefore, such restoration of social dominance makes it possible to prevent and treat a disease related to lack or reduction of social dominance.

According to an example of the present disclosure, AREG exhibits an increased expression level upon activation of CRTC3 by forskolin (FSK), whereas it did not exhibit an increased expression level in CRTC3 KO mice; and a luciferase-expressing gene was inserted into the AREG promoter and activity of AREG was measured, from which it was identified that AREG is a downstream signaling protein of CRTC3 (see Examples 5 and 6).

According to another example, it was identified that deficiency of CRTC3 caused decreased complexity of astrocytes and such an abnormal shape was restored by administration of AREG (see Example 7). In addition, it was identified that CTCR3 KO mice had decreased p-STAT3, which is involved in the AREG signaling pathway, in the cerebral cortex and amygdala as compared with WT mice, and had significantly decreased GluA1, which is known as one of the proteins related to social dominance, in the cerebral cortex (see Example 8).

According to yet another example of the present disclosure, it was identified that in a case where AREG or the EGF domain of AREG (AREG-EGF) was administered to CRTC3 KO mice lacking or having reduced social dominance, social dominance ranking increased. In addition, surprisingly, even in a case where AREG or AREG-EGF was administered to WT mice, which had relatively low social dominance ranking in a WT mouse group, social dominance ranking of the WT mice increased (see Example 9). These results were the same even in a case where AREG was specifically expressed in astrocytes of CRTC3 KO mice (see Example 10). Therefore, activation of EGF receptor with AREG, in particular, the EGF domain of AREG, allows impaired social dominance to be restored.

According to still yet another example of the present disclosure, it was identified that functional connectivity between prefrontal cortex (PFC) and parietal cortex (PC) was significantly reduced in CRTC3 KO rats or mice lacking social dominance (see Example 11). This shows that impairment of social dominance, which is caused by deficiency of CRTC3, is also caused by decreased functional connectivity between PFC and PC, and the functional connectivity can also be restored by administration of AREG.

According to an aspect of the present disclosure, there is provided a pharmaceutical composition for preventing or treating a disease (for example, a psychiatric disease) related to lack or reduction of social dominance, the composition comprising as an active ingredient an agonist for epidermal growth factor (EGF) receptor in the brain. In addition, there is provided a method for preventing or treating a disease related to lack or reduction of social dominance, comprising a step of administering to an individual a composition comprising as an active ingredient an agonist for EGF receptor in the brain.

According to the above-described examples, lack of social dominance in an animal can be restored by amphiregulin (AREG), in particular, the EGF domain of AREG. Therefore, the agonist for EGF receptor in the brain, which is activated by the EGF domain of AREG, is capable of exerting functions equivalent to those of amphiregulin related to social dominance.

In an embodiment, the agonist for EGF receptor may comprise amphiregulin, a fragment thereof, or a nucleic acid encoding the same. In addition, the amphiregulin or the fragment thereof may comprise the EGF domain of amphiregulin.

As used herein, the term "amphiregulin (AREG)" refers to a protein synthesized as type 1 transmembrane glycoprotein and is also called pro-AREG (Non-Patent Document 7). AREG consists of an N-terminal pro-region, a heparin-binding domain (HB), an epidermal growth factor (EGF)-like domain, a transmembrane domain (TM), and a C-terminus. The N-terminal portion of pro-AREG contains two consecutive Lys-Arg-Arg-Arg motifs known to be associated with the nucleus, and these motifs are known as nuclear targeting signals. A soluble form of AREG caused by ectodomain shedding of AREG is produced by proteolytic activity of ADAM-17/TACE that is a transmembrane protease. pro-AREG or cleaved AREG is capable of transmitting signals in a different way. It is capable of transmitting signals by intracellular nuclear translocation or inclusion in exosomes, in juxtacrine, autocrine, and paracrine fashions. AREG is well known as one of the ligands of EGF receptor (EGFR). Activation of EGFR by AREG regulates activation of downstream receptors such as Ras/MAPK, PI3K/AKT, mTOR, STAT, PLCγ, and multiple intracellular signaling pathways.

In another embodiment, the amphiregulin may comprise, but is not limited to, the amino acid sequence represented by SEQ ID NO: 8, and it comprises a sequence having identity of 80%, 81%, 82%, 83%, 84%, 85%, 86%, 87%, 88%, 89%, 90%, 91%, 92%, 93%, 94%, 95%, 96%, 97%, 98%, or 99% with the sequence of SEQ ID NO: 8.

In yet another embodiment, the EGF domain of the amphiregulin may comprise, but is not limited to, the amino acid sequence represented by SEQ ID NO: 10, and it comprises a sequence having identity of 80%, 81%, 82%, 83%, 84%, 85%, 86%, 87%, 88%, 89%, 90%, 91%, 92%, 93%, 94%, 95%, 96%, 97%, 98%, or 99% with the sequence of SEQ ID NO: 10.

Table 3 provides exemplary DNA (or ORF of mRNA) and protein sequences of human amphiregulin, and exemplary DNA and protein sequences of EGF domain of human amphiregulin, including SEQ ID NO: 8 and SEQ ID NO: 10.

[Table 3]

| Item | Type | Sequence | Sequence listing |
|---|---|---|---|
| Human AREG | DNA | ATGAGAGCCCCGCTGCTACCGCCGGCGCC GGTGGTGCTGTCGCTCTTGATACTCGGCT CAGGCCATTATGCTGCTGGATTGGACCTC AATGACACCTACTCTGGGAAGCGTGAACC ATTTTCTGGGGACCACAGTGCTGATGGAT TTGAGGTTACCTCAAGAAGTGAGATGTCT TCAGGGAGTGAGATTTCCCCTGTGAGTGA AATGCCTTCTAGTAGTGAACCGTCCTCGG GAGCCGACTATGACTACTCAGAAGAGTAT GATAACGAACCACAAATACCTGGCTATATT GTCGATGATTCAGTCAGAGTTGAACAGGT AGTTAAGCCCCCCAAAACAAGACGGAA | SEQ ID NO: 7 |
| | | AGTGAAAATACTTCAGATAAACCCAAAAG AAAGAAAAAGGGAGGCAAAAATGGAAA AAATAGAAGAAACAGAAGAAGAAAAAT CCATGTAATGCAGAATTTCAAAATTTCTGC ATTCACGGAGAATGCAAATATATAGAGCA CCTGGAAGCAGTAACATGCAAATGTCAGC AAGAATATTTCGGTGAACGGTGTGGGGAA AAGTCCATGAAAACTCACAGCATGATTGA CAGTAGTTTATCAAAAATTGCATTAGCAG CCATAGCTGCCTTTATGTCTGCTGTGATCC TCACAGCTGTTGCTGTTATTACAGTCCAG CTTAGAAGACAATACGTCAGGAAATATGA AGGAGAAGCTGAGGAACGAAAGAAACTT CGACAAGAGAATGGAAATGTACATGCTAT AGCA | |

(continued)

| Item | Type | Sequence | Sequence listing |
|------|------|----------|------------------|
| Human AREG | Protein | SVRVEQVVKPPQNKTESENTSDKPKRKKK GGKNGKNRRNRKKKNPCNAEFQNFCIHGE CKYIEHLEAVTCKCQQEYFGERCGEKSMK THSMIDSSLSK | SEQ ID NO: 8 |
| Human AREG-E GF | DNA | AAGAAAAATCCATGTAATGCAGAATTTCA AAATTTCTGCATTCACGGAGAATGCAAAT ATATAGAGCACCTGGAAGCAGTAACATGC AAATGTCAGCAAGAATATTTCGGTGAACG GTGTGGG | SEQ ID NO: 9 |
| Human AREG-E GF | Protein | KKNPCNAEFQNFCIHGECKYIEHLEAVTCK CQQEYFGERCG | SEQ ID NO: 10 |

In still yet another embodiment, the disease related to lack or reduction of social dominance may be caused by impaired expression or decreased activity of CRTC3. As used herein, the term "lack or reduction of social dominance" refers to lack or reduction in ability to form and express social hierarchy, and is used as a concept including "social withdrawal." Specifically, the disease related to lack or reduction of social dominance includes both diseases in which lack or reduction of social dominance occurs and diseases caused by lack or reduction of social dominance. In yet another embodiment, the disease related to lack or reduction of social dominance may be caused by impaired expression or decreased activity of CREB-regulated transcription coactivator 3 (CRTC3). In addition, animals lacking or having reduced social dominance may be exposed to social defeat stress, or may experience social defeat stress due to lack or reduction of social dominance. Therefore, in another embodiment of the present disclosure, the disease may be a disease related to social defeat stress. Specifically, the disease related to social defeat stress includes both diseases in which social defeat stress occurs and diseases caused by social defeat stress. The disease in which lack or reduction of social dominance occurs or the disease caused by lack or reduction of social dominance may be a disease accompanied by or caused by social defeat stress.

In still yet another embodiment, the disease may be chronic inflammation. It is known that social dominance ranking influences expression of immune genes, and in particular, low social ranking leads to upregulation of genes involved in innate immune defense and inflammation (see Lea AJ, Akinyi MY, Nyakundi R, Mareri P, Nyundo F, Kariuki T, Alberts SC, Archie EA, Tung J. Dominance rank-associated gene expression is widespread, sex-specific, and a precursor to high social status in wild male baboons. Proc Natl Acad Sci U.S.A. 2018 Dec 26;115(52):E12163-E12171). Therefore, the social defeat stress caused by low social dominance ranking causes chronic inflammation, and such chronic inflammation is a risk factor for aging and disease, which is also associated with shortened lifespan and development of various diseases.

In still yet another embodiment, the disease may be anti-social personality disorder, including self-harming and suicidal ideation. In particular, social defeat stress may cause a mental condition such as self-harming and suicidal ideation, and may lead to a disease such as antisocial personality disorder (see Wolke D, Lereya ST. Long-term effects of bullying. Arch Dis Child. 2015 Sep;100(9):879-85. doi: 10.1136/archdischild-2014-306667. Epub 2015 Feb 10; Mueller AS, Abrutyn S, Pescosolido B, Diefendorf S. The Social Roots of Suicide: Theorizing How the External Social World Matters to Suicide and Suicide Prevention. Front Psychol. 2021 Mar 31;12:621569; Halpern J, Jutte D, Colby J, Boyce WT. Social dominance, school bullying, and child health: what are our ethical obligations to the very young? Pediatrics. 2015 Mar;135 Suppl 2(Suppl 2):S24-30; and the like).

In still yet another embodiment, the disease may be chronic stress. In previous studies, it has been reported that there is a significant association between social dominance and responses to chronic stress, and in particular, male animals with low social dominance ranking show more anxious behaviors in high-stress situations (see Karamihalev S, Brivio E, Flachskamm C, Stoffel R, Schmidt MV, Chen A. Social dominance mediates behavioral adaptation to chronic stress in a sex-specific manner. Elife. 2020 Oct 9;9:e58723).

In still yet another embodiment, the disease may be aggressive personality, anxiety neurosis, schizophrenia, mood disorder, mania, depressive disorder, or bipolar disorder. In particular, it is known that in a case where a lower class suffers from excessive social defeat stress due to a higher class with high social dominance ranking, the lower class may develop a psychiatric disease such as aggressive personality, anxiety neurosis, schizophrenia, mania, depressive disorder, or bipolar disorder (see J Price. The dominance hierarchy and the evolution of psychiatric disease. The Lancet. Vol 290, Issue 7509, 243-246). In addition, it has been reported that serious defects in social dominance behavior appear even in animal models related to schizophrenia and depressive disorder (see Non-Patent Documents 2 and 4).

In still yet another embodiment, the disease may be drug intoxication or mental or behavioral disorder caused by drug intoxication. The social defeat stress makes an animal more sensitive to drugs, and thus an animal lacking or having reduced social dominance may become more easily addicted to drugs.

In still yet another embodiment, the disease may be fragile X syndrome. The phenotypic characteristic of fragile X syndrome is known as lack of social dominance or social withdrawal (see Non-Patent Document 5).

In still yet another embodiment, the disease may be autism spectrum disorder or autism. According to previous studies, animal models of autism and autism spectrum disorder show severe defects in social dominance behavior and also show lack of social interaction (see Non-Patent Documents 2 and 6).

Therefore, the pharmaceutical composition of the present disclosure may be used to prevent or treat various diseases and conditions related to social defeat stress caused by lack or reduction of social dominance (that is, in which social defeat stress is induced or which is caused by social defeat stress), as described above. In addition, according to the methods of the present disclosure, it is possible to prevent or treat a disease related to lack or reduction of social dominance by administering to an individual a prophylactically or therapeutically effective amount of an agonist for EGF receptor in the brain.

Meanwhile, in order to prepare the pharmaceutical composition of the present disclosure, the agonist for EGF receptor in the brain, amphiregulin, a fragment thereof, or a nucleic acid encoding the same may be mixed with a pharmaceutically acceptable carrier and/or excipient. The pharmaceutical composition may be prepared in the form of a lyophilized preparation or aqueous solution. See, for example, Remington's Pharmaceutical Sciences and U.S. Pharmacopeia: National Formulary, Mack Publishing Company, Easton, PA (1984).

The acceptable carriers and/or excipients (including stabilizers) are non-toxic to individuals at the dosages and concentrations used and include, but not limited to, buffers (for exmaple, phosphate, citrate or other organic acids); antioxidants (for exmaple, ascorbic acid or methionine); preservatives (for exmaple, octadecyldimethylbenzyl ammonium chloride; hexamethonium chloride; benzalkonium chloride, benzethonium chloride; phenol, butyl, or benzyl alcohol; alkyl parabens such as methyl or propyl paraben; catechol; resorcinol; cyclohexanol; 3-pentanol; and m-cresol); low molecular weight (about 10 or fewer residues) polypeptides; proteins (for exmaple, serum albumin, gelatin, or immunoglobulin); hydrophilic polymers (for example, polyvinylpyrrolidone); amino acids (for exmaple, glycine, glutamine, asparagine, histidine, arginine, or lysine); monosaccharides, disaccharides, and other carbohydrates such as glucose, mannose, or dextrin; chelating agents (for example, EDTA); sugars (for exmaple, sucrose, mannitol, trehalose, or sorbitol); salt forming counter ions (for example, sodium); metal complexes (for example, Zn-protein complexes); and/or nonionic surfactants (for exmaple, TWEEN (registered trademark), PLURONICS (registered trademark), or polyethylene glycol (PEG)).

The pharmaceutical composition of the present disclosure may be formulated in a suitable form known in the art depending on the route of administration.

As used herein, the term "prophylactically or therapeutically effective amount" or "effective amount" refers to an amount of an active ingredient in a composition, which is effective in preventing or treating a disease related to lack or reduction of social dominance in an individual, the amount being sufficient to prevent or treat the disease at a reasonable benefit/risk ratio applicable to any medical treatment without causing any adverse effects. A level of the effective dose may be determined depending on factors including the patient's health status, type and severity of disease, activity of the drug, sensitivity to the drug, administration method, administration time, administration route and excretion rate, duration of treatment, and drugs used in combination or concurrently therewith, and other factors well known in the medical field. Here, taking all of the above factors into consideration, it is important to administer a minimum amount that allows the maximum effect to be obtained with minimal or no adverse effects, and such an amount may be easily determined by those skilled in the art.

Specifically, the effective amount of the active ingredient in the pharmaceutical composition of the present disclosure may vary depending on age, gender, body weight of the individual (patient), type and severity of disease, form of drug, route and period of administration, and the like, and may be selected appropriately by a person skilled in the art. In general, the effective amount may be a daily dose of about 0.01 to 200 mg/kg, specifically 0.1 to 200 mg/kg, and more specifically 0.1 to 100 mg/kg; however, the scope of the present disclosure is not limited thereto. Administration may be done once a day or in divided doses several times a day, and may also be done over a long period of time by implantation of an infusion pump or the like.

The composition according to the present disclosure can be administered to a subject through various routes. Any mode of administration may be contemplated. The administration may be, for example, parenteral administration, which means administration via injection (for example, bolus injection) or infusion. The parenteral administration includes subcutaneous injection, intravenous injection, intramuscular injection, intraarterial injection, intraperitoneal injection, intracerebral injection, intrathecal injection, or intracerebroventricular injection. The composition according to the present disclosure is preferably administered by intracerebral injection or intracerebroventricular injection.

**Food composition and health functional food composition**

According to still yet another aspect of the present disclosure, there is provided a food composition for preventing or ameliorating a disease related to lack or reduction of social dominance, comprising as an active ingredient an agonist for epidermal growth factor (EGF) receptor in the brain.

The food composition of the present disclosure may also be health functional food.

As used herein, the term "health functional food" refers to food manufactured and processed using raw materials or ingredients with functional properties useful to a human body, which has beneficial effects in preventing and ameliorating a disease related to lack or reduction of social dominance in the present disclosure.

In a case where the agonist for EGF receptor in the brain, or amphiregulin or a fragment thereof, of the present disclosure, is used as a food additive, the agonist, or amphiregulin or a fragment thereof may be added as is or used together with other food or food ingredients, which may be used appropriately according to conventional methods. The amount of the active ingredient to be mixed may be appropriately determined depending on the intended use (prevention, health, or therapeutic treatment). In general, in a case of manufacturing food or beverage, the agent, or amphiregulin or a fragment thereof may be added in an amount of 15% by weight or less, or 10% by weight or less, based on the raw material. However, in a case of long-term intake for the purpose of health and hygiene or health control, the amount may be below the above-mentioned range. The active ingredient may be also used in an amount above the above-mentioned range because there is no problem in terms of safety.

The type of food is not particularly limited. Examples of the food to which the above substances may be added include meat, sausage, bread, chocolate, candy, snacks, confectionery, pizza, instant noodles, other noodles, gum, dairy products including ice cream, various soups, beverage, tea, drinks, alcoholic beverage, and vitamin complexes, in which all health functional food in the conventional sense is included.

The health drink composition according to the present disclosure may comprise various flavoring agents or natural carbohydrates as additional ingredients, like conventional drinks. The above-mentioned natural carbohydrates include monosaccharides such as glucose and fructose, disaccharides such as maltose and sucrose, polysaccharides such as dextrin and cyclodextrin, and sugar alcohol such as xylitol, sorbitol, and erythritol. For the flavoring agent, thaumatin, stevia extract, or the like may be used.

In addition to the above-mentioned ingredients, the composition of the present disclosure may comprise various nutrients, vitamins, electrolytes, flavors, colorants, pectic acid and a salt thereof, alginic acid and a salt thereof, organic acids, protective colloidal thickeners, pH adjusters, stabilizers, preservatives, glycerin, alcohol, carbonating agents used in carbonated drinks, and the like. In addition, the composition of the present disclosure may comprise fruit flesh for natural fruit juice, fruit juice drinks, and vegetable drinks. These ingredients may be used alone or in combination thereof. A ratio of these additives is not very important and is generally selected in a range of 0.01 to 0.20 parts by weight per 100 parts by weight of the composition of the present disclosure.

For a detailed description of the rest parts other than those related to the above-mentioned food composition or health functional food composition, see the section "Pharmaceutical composition."

Hereinafter, the present disclosure will be described in more detail by way of the following examples. However, the following examples are only for illustrating the present disclosure, and the scope of the present disclosure is not limited thereto.

**Examples**

**I. Animal model lacking or having reduced social dominance: CRTC3 KO mice**

**Example 1. Identification of expression of CRTC3 in brain**

**Example 1.1. Identification of region where CRTC3 is expressed in brain**

It is well known that CRTC1 and CRTC2 are mainly expressed in the brain. However, no research has been conducted on expression of CRTC3 in the brain. Accordingly, the present inventors investigated whether CRTC3 was expressed in the brain. The wild type (WT) mouse brain was frozen-sectioned as described in Experimental Example 1.5, stained using an antibody that specifically recognizes CRTC3, and various regions of the brain were examined to check the locations where CRTC3 is expressed.

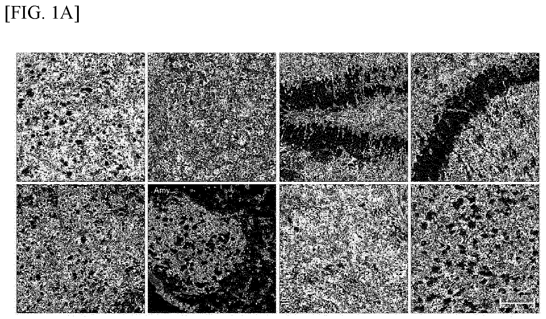

As a result, it was identified that CRTC3 was expressed in the prefrontal cortex (PFC), cingulate cortex (Cg), hippocampus, dentate gyrus (DG), cornu ammonis (CA) 3 region, hypothalamus (Hy), amygdala (Amy), ventral tegmental area (VTA), and medial dorsal nucleus (MD) (FIG. 1A). It was identified that in addition to the brain regions shown in FIG. 1A, CRTC3 was also expressed in the olfactory bulb, cerebellum, and the like (data omitted). These experimental results demonstrate that CRTC3 is expressed in various regions of the brain.

**Example 1.2. Identification of cells expressing CRTC3**

Next, double staining was performed to identify cell types expressing CRTC3. NeuN (neurons), GFAP (astrocytes), s100β (astrocytes), and Iba1 (microglia) were used for staining which are markers that allow identification of major cells in the brain.

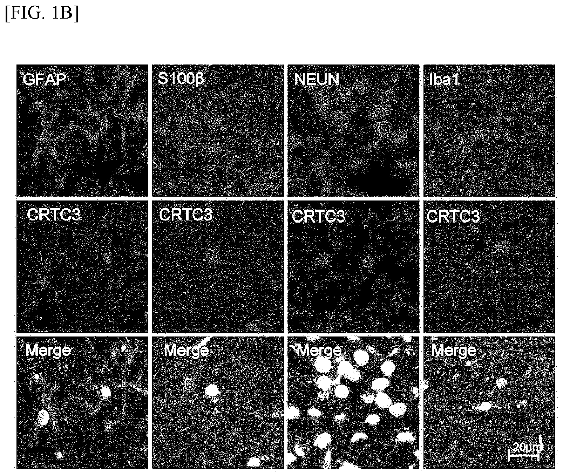

The experimental results showed that CRTC3 co-localized with GFAP (FIG. 1B). These results mean that CRTC3 is expressed exclusively in astrocytes, not glial cells and neurons (DIV 14).

To cross-validate the above results, each cell type was primary cultured and a level of CRTC3 mRNA therein was checked by qRT-PCR.

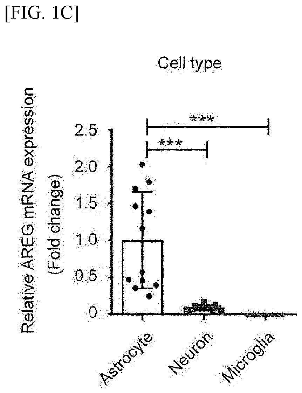

It was identified that consistent with the above results, the primary astrocytes showed the highest expression level of CRTC3 mRNA and the microglia hardly expressed CRTC3 mRNA (FIG. 1C).

The experimental results of Example 1 show that CRTC3 is expressed in various regions of the brain, and in particular, is mainly expressed in astrocytes among various cells in the brain.

**Example 2. Production of CRTC3 KO mice**

**Example 2.1. Laboratory animal**

CRTC3 knockout (KO) mice were obtained from Ulsan University. The CRTC3 KO mice were produced as described in Song Y, Altarejos J, Goodarzi MO, Inoue H, Guo X, Berdeaux R, Kim JH, Goode J, Igata M, Paz JC, Hogan MF, Singh PK, Goebel N, Vera L, Miller N, Cui J, Jones MR; CHARGE Consortium; GIANT Consortium, Chen YD, Taylor KD, Hsueh WA, Rotter JI, Montminy M. CRTC3 links catecholamine signaling to energy balance. Nature. 2010 Dec 16;468(7326):933-9. Specifically, CRTC3$^{-/-}$ mice carrying a mutant CRTC3 allele (that is, with deletion of exon 1 encoding CREB-binding domain (CBD)) were produced by homologous recombination using a CRTC3 targeting vector containing a Neo selection marker instead of exon 1 encoding CBD. The targeting vector was constructed by replacing exon 1 of the CRTC3 gene encoding CBD with a phosphoglycerol kinase (pgk)-neomycin selection cassette. In addition, the targeting vector contains a pgk-diphtheria toxin A cassette for negative selection. The vector constructed as above was linearized, and then introduced into R1 embryonic stem cells by electroporation. G418-resistant clones were screened for homologous recombination using Southern blotting. The CRTC3$^{-/-}$ mice were backcrossed to C57/BL6 mice for up to three generations for this study.

To compare the CRTC3 KO mice with wild type (WT) mice, two to three pairs of siblings were taken after birth and housed together in cages. The respective mice in a sibling group were randomly selected. Twelve-week-old male C57BL/6 mice were purchased from KOATECH (Seoul, Korea). The C57BL/6 mice were housed together in cages since birth of three pairs of siblings. Each sibling group of the mice was housed in a different cage, had free access to water and food, and the surrounding environment was maintained at a constant temperature (22 ± 1°C) with a light/dark cycle of 12:12 hours.

All animal experimental procedures were performed with approval from the Ethics Committee for Animal Experiments of the Asan Institute for Life Sciences (Seoul, Korea)

**Example 2.2. Genotyping of experimental animals**

For the WT mice and CRTC3 KO mice, genomic DNA was obtained therefrom by tail biopsy, and then genotyping was performed using the primer sets shown in Table 4 to finally identify their genotype.

[Table 4]

| Allele | Orientation of primer | Sequence | Sequence listing |
|---|---|---|---|
| CRTC3 WT | Forward | 5'-CCTGAGTTATTGGCGGATGT-3' | SEQ ID NO: 15 |
| | Reverse | 5'-CACTCAGGCTGTAGCAAGCA-3' | SEQ ID NO: 16 |
| CRTC3 KO | Forward | 5'-ATGGAAGGATTGGAGCTACG-3' | SEQ ID NO: 17 |
| | Reverse | 5'-CACTCAGGCTGTAGCAAGCA-3' | SEQ ID NO: 18 |

**Example 3. Identification of reduced social dominance in CRTC3 KO mice**

To determine what role CRTC3 plays in the brain, a series of behavioral tests were performed using the WT and CRTC3 KO mice. First, a tube dominance test, a warm spot test, and a urine marking test were performed to evaluate social dominance (or social preponderace, social ranking) for the experimental animals (FIG. 2A to 2M).

**Example 3.1. Identification of reduced social dominance in CRTC3 KO mice through tube dominance test**

**Example 3.1.1. Tube dominance test method**

The tube dominance test is a standard test that allows determination of dominance ranking according to social hierarchy in rodents. In addition to this test, various types of behavioral tests are known which allow determination of social dominance. The tube test is advantageous in that it can be performed relatively safely without exposing mice to strenuous conditions. In addition, the tube test can be modified in various ways and can be safely performed in the same cage or different cages, which allows various test data to be obtained. This tube test is also advantageous in that it is a very simple test for which the essential apparatus required is a narrow cylindrical tube through which a mouse can pass.

The apparatus for the tube dominance test was made of a transparent plastic tube with a length of 30 cm and an inner diameter of 2.5 cm. To allow the mice to adapt to passing through the inside of the tube, the respective mice were trained to pass across the tube alternately on both sides for two or three consecutive days. During the training period, care was taken to ensure that the mice passed through the tube as naturally as possible without causing stress. This test was performed after identifying that all mice could freely pass through the tube without stress.

During the dominance test period, a total of 2 mice, one at each end of the tube, were placed to meet in the middle of the tube. In a case where one mouse pushed the other mouse out of the tube, one (1) winning point was earned. The test was repeated three times. For example, in a case where one mouse earned 2 or 3 winning points, the mouse was considered a winner and the other mouse was considered a loser. For the mice housed in the same cage, the tube test was performed on three mice in turn to determine social dominance ranking. For example, for mouse no. 1, the tube test was performed with mouse nos. 2 and 3. In a case where mouse no. 1 wins both mice, it is ranked first. Meanwhile, in a case where mouse no. 2 loses to mouse no. 1 and wins mouse no. 3, it is ranked second. In the two tests, mouse no. 3 is ranked third since it loses to both mice. For each test, win or loss was determined by winning points.

In the present example, the tube dominance test was performed as follows. First, after habituation, the WT and CRTC3 KO mice were placed in the same cage at both ends of the tube, respectively, and were allowed to meet in the middle of the tube. In a case where the two mice did not meet each other in the middle of the tube, the test was repeated. The tube test ended in a case where the two mice encountered each other in the tube and one mouse pushed the other mouse out of the tube (FIG. 2A). The test time did not exceed 2 minutes. In a case where the test time exceeded 2 minutes, the test was performed again. The mouse that pushes the other mouse out of the tube wins and earns 1 winning point. Conversely, the mouse that is pushed out of the tube loses. These two mice underwent a total of three tube tests, and the mouse that earned 2 or higher winning points was considered the final winner. To identify the social dominance ranking (social ranking) within the cage of CRTC3 KO mice, the tube dominance test was performed for 5 days.

Meanwhile, the tube was sterilized with 70% ethanol before starting each test.

### Example 3.1.2. Tube dominance test results

In the tube dominance test, male CRTC3 KO mice showed significant changes in their behavior (FIGS. 2B to 2F).

As compared with the WT mice, the CRTC3 KO mice had significantly lower winning points (FIG. 2B). Even from the results obtained by comparing the number and duration of push-initiated and push-back between the WT mice and the CRTC3 KO mice, it was identified that the CRTC3 KO mice had lower social dominance (FIGS. 2C to 2F).

In addition, to compare the social dominance between the WT mice and the CRTC3 KO mice, the test was performed in two modes.

In the first mode, the test was performed using sibling mice housed in the same cage (left graph of FIG. 2G, Familiar group). Specifically, in a case where the mice were divided into each cage, the WT and CRTC3 KO mice were housed together and raised. For the WT and CRTC3 KO mice raised in the same cages, the tube test was performed in all cases. The test for the WT and CRTC3 KO mice was performed in 25 cages, in which 84% of the WT mice won and 16% of the CRTC3 KO mice won. That is, the CRTC3 KO mice showed a significantly decreased number of wins as compared with the WT mice (WT, n = 50; CRTC KO, n = 28; tube test, n = 56; ***p < 0.0001).

In the second mode, social ranking was compared between the WT mice and the CRTC3 KO mice which were housed separately in different cages (right graph in FIG. 2G, Unfamiliar group). Even in a case of being housed in different cages, 69% of the WT mice and 31% of the CRTC3 KO mice won, indicating that the CRTC3 KO mice showed a significantly decreased number of wins as compared with the WT mice (WT, n = 12; CRTC3 KO, n = 11; tube test, n = 55). As a result, the CRTC3 KO mice showed low ranking as compared with the WT mice.

Meanwhile, to rule out the possibility that differences in social dominance were caused by differences in mouse body size, tube tests were performed on male mice of the same weight. Even in the tests with the mice matched to the same weight, the CRTC3 KO mice showed low social ranking (FIG. 2H).

Additionally, it was identified whether CRTC3 affects social dominance ranking even in female mice in the same way as in the male mice. Even for the female mice, the tube dominance test was performed in the same two modes as in the male mice.

As a result, it was identified that the female CRTC3 KO mice showed significantly low social dominance ranking as compared with the female WT mice in both the group where the female WT mice and the female CRTC3 KO mice were raised in the same cage (FIG. 2I) and the group where the female WT mice and the female CRTC3 KO mice were raised in different cages (FIG. 2J). These results demonstrate that CRTC3 plays an important role in formation of dominant social ranking not only in male mice but also in female mice.

### Example 3.2. Identification of reduced social dominance in CRTC3 KO mice through warm spot test

### Example 3.2.1. Warm spot test method

The warm spot test was performed using the following apparatus and method. The test apparatus was a box with dimensions of 30 cm by 30 cm, and the bottom was kept cold by ice. An fan-shaped heating mat of approximately 3 cm was laid one corner of the bottom to warm only that part of the bottom. In the first test, the mice were placed in a box with all bottom surfaces cold and kept thereon for 30 minutes to get used to the environment. After 30 minutes, the mice were moved to a box with a heating mat on one corner of the bottom, and the time each mouse occupied the bottom surface with the heating mat was measured for 20 minutes. Based on the measured time, each mouse's ranking in the group was determined.

### Example 3.2.2. Warm spot test results

From the results obtained by performing the warm spot test on the WT mice and the CRTC3 KO mice, the time for the CRTC3 KO mice to occupy the warm spot appeared to be significantly shorter than the time for the WT mice to occupy the warm spot. Accordingly, consistent with the tube dominance test results, the CRTC3 KO mice were evaluated as having low dominance ranking even in the warm spot test (FIG. 2K).

### Example 3.3. Identification of reduced social dominance in CRTC3 KO mice through urine marking test

### Example 3.3.1. Urine marking test method

It is known that in a urine marking test, mice with dominant social ranking make urine marking more frequently and more abundantly. The urine marking test allows determination of social ranking of the mice, like the tube dominance test and the warm spot test.

The urine marking test was performed as follows. First, filter paper was laid in a cage with an area of 28 cm × 6.5 cm, and a wire mesh was installed in the middle of the cage to separate the sections. Here, the mouse placed in each section was allowed to touch or sniff. The WT mouse and the CRTC3 KO mouse raised in the same cage were placed in separate sections and then observed for 2 hours. After 2 hours, a ninhydrin solution was sprayed on the filter paper and checked by UV The area where ninhydrin reaction occurred was marked on an OHP film having a grid of 0.4 mm × 0.4 mm in size. The grid area of the urine spot was calculated and converted into data, and the test was performed with a pair of one WT mouse and one CRTC3 KO mouse per day. For example, in a case where one WT mouse and two CRTC3 KO mice were raised together, the test was performed for a total of 2 days in such a way that the WT mouse and one of the CRTC3 KO mice were tested once a day.

**Example 3.3.2. Urine marking test results**

From the results obtained by performing the urine marking test on the WT mice and the CRTC3 KO mice, it was found that the CRTC3 KO mice had significantly smaller urine marking spot areas than the WT mice (FIGS. 2L and 2M). In other words, it was identified through urine marking spots that the CRTC3 KO mice showed lower social ranking than the WT mice.

The test results of Examples 3.1 to 3.3 show that animals lacking CRTC3 in the brain have low social dominance.

**Example 4. Identification of behavioral changes other than social dominance in CRTC3 KO mice**

To identify other behavioral changes seen in the CRTC3 KO mice, various behavioral tests were performed (FIGS. 3A to 3D). Specifically, a Y-maze test and a Morris water maze test were performed to evaluate the long-term and short-term memory of experimental animals. In addition, an olfactory preference test and a novel object recognition test were performed to evaluate behavioral changes related to senses such as smell and vision in experimental animals.

**Example 4.1. Identification of impaired short-term memory in CRTC3 KO mice**

**Example 4.1.1. Y-maze test method**

The Y-maze test is a test of short-term memory performed in a horizontal maze (10 cm × 50 cm × 20 cm) with three arms, each arm of the maze symmetrically spaced at an angle of 120°. To test short-term memory, in the first session, one arm of the maze was closed and the other two arms were open. The mouse was placed in the center of the maze and allowed to freely explore the two open arms for 10 minutes. In the test session performed after three hours, the closed arm was opened so that the mouse could explore the new arm. The mouse was placed in the center of the maze and allowed to freely explore the three open arms for 5 minutes. The test was measured and recorded by a smart video tracking system (software version 3.0, Panlab, Harvard Apparatus, USA). Data on the time of entry and the number of entries into the newly opened arm were collected and analyzed. Before start of each test, the maze was washed using 70% ethanol to remove residual odor.

**Example 4.1.2. Y-maze test results**

As described in Example 4.1.1, initially one of the three arms was blocked, and the mouse was allowed to freely move only the remaining two arms. After 2 hours, one blocked arm was opened, and the number of times the mouse entered the new arm and the time it stayed in the new arm were measured. The Y-maze test results showed no significant differences between the WT mice and the CRTC3 KO mice (FIG. 3A).

**Example 4.2. Identification of impaired long-term memory in CRTC3 KO mice**

**Example 4.2.1. Morris water maze test method**

The Morris water maze test for testing long-term memory was performed using a circular swimming pool (height 50 cm × diameter 120 cm, water temperature of 24 ± 1°C). For the test, a circular escape platform with a diameter of 10 cm was placed 1.5 cm below the water surface in the center of the NW quadrant. The swimming pool was filled to a depth of 40 cm with liquid made by dissolving skim milk in water, and the liquid was replaced with fresh liquid every day. Additional maze cues consisted of geometric shapes of the walls, posters, and furniture of the room where the pool was located. One cue was attached to the wall of the water maze pool near the escape platform.

The test procedure was conducted over a total of 5 testing days consisting of 4 training test days and 1 probe test day. During the training test, the mouse was placed to face the wall in the third quadrant and allowed to find the escape platform for 1 minute. In a case where the mouse found the escape platform within the designated time, it was allowed to stay there for 1 minute. Otherwise, the mouse was guided to the escape platform and was allowed to stay there for 1 minute. Each mouse was given three training sessions with 1 minute of rest between the respective training sessions. In the three training sessions, the mouse was placed in different quadrants (NE, SE, and SW) where the escape platform was not located. After the training sessions, the probe test was performed on the mouse without the escape platform. The mouse was placed to face the wall in the SE quadrant, and the platform was fine-tuned. Every test was measured and recorded by a smart video tracking system (software version 3.0, Panlab). Data were collected as swimming time (s) and swimming distance (cm) to reach the escape platform, and analyzed.

**Example 4.2.2. Morris water maze test results**

As described in Example 4.2.1, during the training test days, the mice were placed in the pool and trained to find the escape platform. The training test was performed for 4 days. The next day, the escape platform was removed and the time, during which each mouse stayed in the place where the removed platform had been located, was measured.
As a result, the CRTC3 KO mice showed no difference in terms of long-term memory as compared with the WT mice (FIG. 3B, Time in platform). In addition, even during the 4 training test days, the WT mice and the CRTC3 KO mice traveled similar distances to find the platform (FIG. 3B, Distance to target). Meanwhile, on day 1 of the training test, the CRTC3 KO mice seemed to find the platform in a shorter time than the WT mice; however, no difference between the CRTC3 KO mice and the WT mice was seen during the remaining 3 days of training test (FIG. 3B, Latency to target). In the probe test, comparison of the time taken to find the removed escape platform was performed between the CRTC3 KO mice and the WT mice. As a result, it was identified that both CRTC3 KO mice and WT mice spent a similar amount of time to find the removed platform and also showed no difference in terms of the time staying in the area where the platform had been located (FIG. 3B).

**Example 4.3. Identification of sensory-related behavioral changes in CRTC3 KO mice**

**Example 4.3.1. Olfactory preference test method**

For the olfactory preference test, four empty cages, 10% 2-methylbutyric acid (2-MBA), and 10% peanut butter solution were prepared. The mice were habituated to the four cages in order for 15 minutes each. After 15 minutes had elapsed in the fourth cage, a filter paper soaked with a diluted scent solution was placed on the other side of the mouse and video was recorded for 3 minutes. After 3 minutes, the filter paper was removed, and recording was performed in the same manner as above using a filter paper soaked with a different scent to measure the time for the mouse to approach the filter paper and show interest thereon.

**Example 4.3.2. Novel object recognition test method**

The new object recognition test was performed by the following procedure. First, models of the same shape were placed at both ends of a $40 \times 40$ cm box and each mouse was allowed to explore them for 15 minutes. After 15 minutes, one model was replaced with a model with a different color and shape, and the time for the mouse to approach and explore the new model was measured over a 15-minute observation period.

**Example 4.3.3. Results of olfactory preference test and novel object recognition test**

An olfactory preference test and a new object recognition test were performed according to the methods described in Example 4.3.1 and Example 4.3.2 to determine whether sensory-related behavioral changes occurred in the CRTC3 KO mice.
In the olfactory preference test, the CRTC3 KO mice showed no difference as compared with the WT mice (FIG. 3C). Both the CRTC3 KO mouse group and the WT mouse group showed high preference for peanut butter and tended to avoid 2-methylbutiric acid (2-MBA).
As a result of measuring the time for each mouse to respond to a new object through the novel object recognition test, it was identified that the CRTC3 KO mice showed a similar interest level to the WT mice (FIG. 3D).
The test results of Examples 4.1 to 4.3 show that lack of CRTC3 in the brain does not cause impaired short-term or long-term memory or changes in sensory-related behavior.
Summarizing the results of the above examples, while the CRTC3 KO mice showed significantly decreased social dominance as compared with the WT mice, they showed no change in long-term memory, short-term memory, behaviors related to senses, or the like. Therefore, CRTC3 plays an important role in recognizing social dominance in animals; and animals in which expression of this CRTC3 gene is suppressed or reduced or activity of CRTC3 protein is suppressed or reduced can be used as an animal model lacking social dominance, and can also be further used as an animal model for various diseases or conditions related to lack or reduction of social dominance, such as chronic inflammation, self-harming, suicidal ideation, anti-social personality disorder, aggressive personality, chronic stress, anxiety neurosis, drug intoxication or mental or behavioral disorder caused by drug intoxication, psychiatric diseases, including schizophrenia, mood disorder, mania, depressive disorder, bipolar disorder, fragile X syndrome, autism spectrum disorder, and autism.

II. **Use of amphiregulin (AREG) or EGF domain thereof for treatment of diseases**

**Example 5. Identification of AREG as target gene directly regulated by CRTC3**

To identify changes in expression of genes related to CRTC3 deficiency, whole transcript profiling was performed using a mouse cDNA microarray as described in Experimental Example 1.3 (FIG. 4). To identify changes in expression of CRTC3-related genes in cells expressing CRTC3, astrocytes were treated with forskolin (FSK) to activate CRTC3. FSK was purchased from Sigma-Aldrich (St. Louis, USA), and prepared at a concentration of 25 $\mu$M using DMSO and used. FSK dephosphorylates CRTC to induce its nuclear translocation so that expression of the gene is regulated. Fold-change values obtained by comparing samples, which were treated with FSK for 2 hours, with untreated samples were compared between the WT mice and the CRTC3 KO mice. Specifically, genes whose expression increased after treatment with FSK in the WT mice were listed, and the top 29 genes with high expression levels were selected therefrom and compared with those in the CRTC3 KO mice. The 29 genes identified as target genes of CRTC3 are as shown in Table 5.

[Table 5]

| Procr | AREG | Il6 | Penk | Vdr | Thbd |
|---|---|---|---|---|---|
| Cytip | Prg4 | Gprc5a | Has1 | Rasd1 | Gzme |
| Il1b | Dgat1 | Olfr130 | Nefl | Cxcl1 | Zfp273 |
| Trmt61b | Apold1 | Tac1 | Svs3b | Olfr584 | Hist1h4n |
| Olfr1428 | Cyp3a57 | Vmn1r122 | Hist1h4 | Foxq1 | - |

Treatment with FSK increased expression of most genes in the WT mice, but not in the CRTC3 KO mice (FIG. 4A). Differences in expression level of the respective genes were compared between the WT mice and the CRTC3 KO mice, and the 15 genes were sorted therefrom in order from the gene with the highest difference to the gene with the lowest difference (FIG. 4B). Among these, for the genes Procr and AREG, their expression level increased by treatment with FSK in the WT mice, but not in the CRTC3 KO mice (FIG. 4A), and these genes showed the greatest difference upon comparison of gene expression levels between the WT mice and the CRTC3 KO mice (FIG. 4B). In particular, AREG was known to be a protein that is secreted from cells and binds to epidermal growth factor (EGF) receptor and other cell surface receptors to generate signal transduction, and thus it was expected that AREG is involved in regulating social dominance (or social control) in the brain. The experimental results of the present example show that promoted expression of amphiregulin (AREG) caused by FSK is suppressed in astrocytes of the CRTC3 KO mice, and that AREG is a CREB target gene directly regulated by CRTC3.

**Example 6. Identification of expression pattern of AREG and its association with CRTC3**

To investigate association between the target genes of CRTC3 and AREG, various types of cells present in the brain of WT mice were primary cultured and qRT-PCR analysis was performed according to the method described in Experimental Example 1.2.

As a result, it was identified that similar to the expression pattern of CRTC3 identified in Example 1, AREG was mainly expressed in astrocytes, but not in neurons and microglia (FIG. 5A).

In addition, to identify whether expression of AREG was also reduced in the CRTC3 KO astrocytes as shown in the microarray analysis results of Example 5, qRT-PCR was performed according to the method described in Experimental Example 1.2.

As a result, it was identified that the expression level of AREG was significantly lower in astrocytes of the CRTC3 KO mice as compared with primary astrocytes of the WT mice (FIG. 5B). In addition, even in a case where CRTC3 was activated by treatment with FSK, primary astrocytes of the WT mice exhibited an increased expression level of AREG by treatment with FSK, whereas primary astrocytes of the CRTC3 KO mice did not exhibit an increased expression level of AREG despite treatment with FSK (FIG. 5C).

To identify whether expression of AREG is affected by CRTC3, a luciferase assay was performed. A luciferase expression gene was inserted into the AREG promoter, and then activity of luciferase was measured.

As a result, in the cells expressing CRTC3, the AREG promoter was driven to activate luciferase, and luciferase was activated to a greater extent in a case of being treated with FSK (FIG. 5D). On the other hand, it was identified that significantly low luciferase activity was observed in AREG-CRE mutants despite expression of CRTC3 or treatment with FSK. These results support that AREG is a downstream signaling protein of CRTC3.

**Example 7. Identification of shape recovery effect of AREG on astrocytes**

Next, to identify localization of CRTC3 in the cellular changes induced in astrocytes, comparison of astrocyte shape was performed between the CRTC3 KO mice and the WT mice. The shape of the cells was observed using GFAP as an astrocyte marker through fluorescent staining according to the method described in Experimental Example 1.4.

As a result, it was identified that astrocytes in the CRTC3 KO mice were larger in cell size and less complex in shape as compared with astrocytes in the WT mice (FIG. 5E).

To more accurately compare the astrocyte shapes, complexity of the astrocyte shape was determined using the cell shape index (CSI) defined as follows (in the formula, P is a cell's perimeter and A is a cell's area).

$$CSI = \frac{P^2}{4\pi A}$$

Astrocytes are connected to neurons and capillaries to transmit neurotransmitters or induce nerve signals. Therefore, more complex cells have higher CSI.

The astrocytes in the CRTC3 KO mice showed significantly lower CSI values than the astrocytes in the WT mice (FIG. 5F). It was identified whether such changes in CSI were recovered by AREG. As a result, AREG increased a CSI level of the primary astrocytes in the CRTC3 KO mice to that of the WT astrocytes (FIG. 5F). These results demonstrate that AREG restored the abnormally modified astrocyte shape in the CRTC3 KO mice.

The results of Example 7 show that astrocytes whose shape has been damaged due to lack of CRTC3 can be recovered by treatment with AREG.

**Example 8. Identification of decreased expression levels of p-STAT3 and GluA1 in CRTC3 KO mice**

AREG is one of the EGFR ligand family and interacts with EGFR to activate subsequent signaling pathways such as mitogen-activated protein kinase (MAPK), phosphatidylinositol 3-kinase (PI3K)/protein kinase B (Akt), and signal transducer and activator of transcription 3 (STAT3). Therefore, to investigate whether disruption of CRTC3 causes changes in levels of proteins related to AREG signaling and social dominance, Western blotting analysis was performed on the cortex (CTX) and amygdala (AMY) in the CRTC3 KO mouse brain using the method described in Experimental Example 1.6 (FIG. 6).

From the experimental results, it was identified that phosphorylated STAT3, that is, p-STAT3, was significantly decreased in both cortex (CTX) and amygdala (AMY) samples from the CRTC3 KO mouse brain (p < 0.005; FIGS. 6A and 6B). These data are consistent with the results that AREG was reduced in the CRTC3 KO mice, and verify that the expression level of p-STAT3, which is a downstream signaling molecule of the AREG signaling pathway, was also reduced. Meanwhile, it was found that the expression level of STAT3 was increased in the amygdala of the CRTC3 KO mice (FIG. 6B, upper right graph).

According to the previous study, it has been reported that phosphorylation of $\alpha$-amino-3-hydroxy-5-methyl-4-isoxazolepropionic acid (AMPA) receptor is reduced in the medial prefrontal cortex (mPFC) from the mice that lacked social dominance due to stress (see Park MJ, Seo BA, Lee B, Shin HS, Kang MG. Stress-induced changes in social dominance are scaled by AMPA-type glutamate receptor phosphorylation in the medial prefrontal cortex. Sci Rep. 2018 Oct 9;8(1):15008). Another study showed that expression of glutamate receptor 1 (GluA1) and GluA2, which are the subunits of AMPA receptor, is reduced in mPFC of a mouse model of autism spectrum disorder (ASD) that is characterized by impaired social development. In this mouse model, it was shown that administration of AMPA positive-allosteric modulator (PAM) improved the expression level of AMPA receptor and restored social dominance that had lacked (see Kim JW, Park K, Kang RJ, Gonzales ELT, Kim DG, Oh HA, Seung H, Ko MJ, Kwon KJ, Kim KC, Lee SH, Chung C, Shin CY Pharmacological modulation of AMPA receptor rescues social impairments in animal models of autism. Neuropsy-

EP 4 368 196 A1

chopharmacology. 2019 Jan;44(2):314-323).

Based on the above studies, in the present example, it was identified whether the expression level of GluA1, which is the subunit of AMPA receptor, changed in the CRTC3 KO mice. As a result, it was identified that the expression level of GluA1 was significantly reduced in the cortex (FIG. 6A, lower right graph; p = 0.0578), and that the expression level of GluA1 was significantly increased in the amygdala (FIG. 6B, lower right graph; p < 0.005).

In summary, for the CTCR3 KO mice, p-STAT3, which is involved in AREG signaling pathway, was decreased in the cerebral cortex and amygdala, and GluA1, which is one of the proteins that regulate social dominance, was significantly decreased in the cerebral cortex.

The results of Example 8 show that the expression levels of both p-STAT3 and GluA1 in the cerebral cortex (CTX) of the CRTC3 KO mice were significantly reduced as compared with those of the WT mice.

**Example 9. Identification of effect of increasing social dominance ranking by administration of AREG or AREG-EGF in CRTC3 KO mice**

**Example 9.1. Experiment material**

Recombinant mouse AREG and EGF used in the present example were purchased from R&D Systems (Minneapolis, USA). AREG-EGF peptide and AREG-HB peptide were purchased from Peptron Co., Ltd. (Daejeon, Korea). The amino acid sequences of the mouse AREG and EGF and the amino acid sequences of the AREG-EGF and AREG-HB peptides are shown in Table 6.

[Table 6]

| Item | Amino acid sequence | Sequence listing |
|---|---|---|
| Mouse AREG | SVRVEQVIKPKKNKTEGEKSTEKPKRKKK GGKNGKGRRNKKKKNPCTAKFQNFCIHG ECRYIENLEVVTCNCHQDYFGERCGEKSM KTHSEDDKDLSK | SEQ ID NO: 11 |
| Mouse EGF | NSYPGCPSSYDGYCLNGGVCMHIESLDSY TCNCVIGYSGDRCQTRDLRWWELR | SEQ ID NO: 12 |
| AREG-EGF peptide | KKNPCNAEFQNFCIHGECKYIEHLEAVTC KCQQEYFGERCGEK | SEQ ID NO: 13 |
| AREG-HB peptide | SVRVEQVVKPPQNKTESENTSDKPKRKKK GGKNGKNRRNRK | SEQ ID NO: 14 |

Meanwhile, 3.5-month-old CRTC3 KO mice were prepared in the same manner as in Example 1.2.

**Example 9.2. Experimental method**

To determine a role of AREG in the behavioral changes toward lower social dominance ranking which are seen in the CRTC3 KO mice, AREG or AREG-EGF was delivered to the 3.5-month-old CRTC3 KO mice via intracerebroventricular injection (ICV), and changes in social dominance ranking were examined (FIG. 7).

First, starting at 1 month of age, the CRTC3 KO mice and the WT mice were housed in one group and raised together for 2 months. This allowed social ranking to be naturally formed among the mice in the same group. To determine changes in ranking of the mice in the group, social dominance ranking was evaluated by a tube dominance test daily for 15 days starting at 3 months of age. The respective mice in the group were tested in turn, and dominance ranking of the mice in the group was determined based on their winning points. After identifying that social dominance ranking of the mice in the group was maintained constant, each of AREG (FIGS. 7A and 7C), AREG-EGF (FIGS. 7D and 7E), and AREG-HB (FIG. 7F) was administered for 1 month to the lateral ventricle of the right brain using an osmotic pump

32

according to the method described in Experimental Example 2.1. Experiments were performed in two mouse groups: cages where the CRTC3 KO mice and the WT mice are raised together (FIGS. 7A and 7E) or cages where only the C57BL/6 mice are raised (FIGS. 7C, 7D, and 7F). For each cage, the CRTC3 KO mice or C57BL/6 mice with low social dominance ranking received a drug containing AREG, AREG-EGF, AREG-HB, or mEGF, and the WT mice (C57BL/6 mice) with high social dominance ranking received PBS as a control. An osmotic pump was implanted in each mouse according to the method described in Experimental Example 2.1, and then a recovery period of 1 week was given. A tube dominance test was performed every day starting one week after the implantation surgery, and changes in social dominance ranking were observed again.

**Example 9.3. Effect of increasing social dominance ranking by administration of AREG**

The test was performed on 16 cages containing 16 CRTC3 KO mice. As a result, changes in dominance ranking were observed in 13 cages after administration of AREG to the CRTC3 KO mice (13/16, 81%; bar graph in FIG. 7A). In addition, in a case of comparing the winning points before and after the drug administration, the CRTC3 KO mice having received AREG showed an increase in winning points after the administration as compared with before the administration. Even in the warm spot test, which is another test for determining ranking, after the drug administration, the CRTC3 KO mice exhibited an increase in the time they occupied the warm spot, and also exhibited increased social dominance ranking (FIG. 7B). These results show that the low social dominance ranking of the CRTC3 KO mice was increased by administration of AREG.

Next, based on the above test results that administration of AREG can increase the low social dominance ranking of the CRTC3 KO mice, the same tests were performed in cages where only WT mice (= C57BL/6 mice) were grouped to ensure consistency of the results (FIG. 7C). Specifically, 3-month-old C57BL/6 mice were purchased, housed in the same cage, and allowed to adapt to the new environment for 1 week. After one week, the mice were trained to adapt to passing through the tube, and a tube dominance test was performed every day until social dominance ranking of the respective mice in the group was maintained constant. AREG was administered to the mouse with the lowest social ranking, and the same change in its dominance ranking as in the above tests was observed during the drug administration. That is, consistent with the above results that the social dominance ranking of the CRTC3 KO mice was increased by administration of AREG, an effect of increasing the ranking of the lowest-ranked mouse was also observed by administration of AREG in the C57BL/6 mice. This effect of increasing social dominance ranking was observed in 12 of 17 cages (12/17, 71%; bar graph in FIG. 7C).

**Example 9.4. Effect of increasing social dominance ranking by administration of AREG-EGF**

Meanwhile, to identify that the EGF domain in AREG protein is an important factor causing changes in social dominance ranking, social dominance tests were performed respectively in the C57BL/6 mouse group (FIG. 7D) and the WT and CRTC3 KO mouse groups (FIG. 7E).

The test results showed that for the C57BL/6 mouse group, the ranking of the lowest-ranked C57BL/6 mouse was increased by administration of AREG-EGF in 8 out of 10 cages (8/10, 80%; bar graph in FIG. 7D). In addition, even in a case where AREG-EGF was administered to the CRTC3 KO mice with low social dominance ranking, changes in winning points and increases in tube test ranking were observed in 4 out of 8 cages after the administration (4/8, 50%; bar graph in FIG. 7E).

In addition, tests were performed in the same manner as above using HB domain, which is another domain of AREG, and mouse EGF (mEGF) as a control.

In a case where AREG-HB was administered, an increase in social dominance ranking was observed in only one cage (1/10, 10%; FIG. 7F, top graph). For the group having received mEGF, no changes were observed in the social dominance ranking determined before the administration (0/4, 0%; FIG. 7F, bottom graph). These results show that the EGF domain of AREG is an important region that causes changes in dominance ranking.

The results of Examples 9.3 and 9.4 show that social dominance ranking can be increased by administration of AREG or AREG-EGF in both the WT mice and the CRTC3 KO mice which have low social dominance ranking.

**Example 10. Identification of effect of increasing social dominance ranking by astrocyte-specific expression of AREG in CRTC3 KO mice**

To identify whether AREG protein produced in astrocytes causes changes in social ranking of CRTC3 KO mice, the AREG protein was specifically expressed in astrocytes using the AAV5 virus system described in Experimental Example 2.2. For astrocyte-specific expression of the protein, the GFAP promoter system was used, and a construct was made such that amino acids 94-191, which include the EGF domain, of the amino acid sequence of mouse AREG were synthesized after the signal sequence.

Since the EGF domain of AREG was found to be an important domain in changing social dominance ranking of the CRTC3 KO mice in Example 9, the EGF domain of AREG was specifically expressed in astrocytes. The virus thus produced was injected into the right ventricle of the CRTC3 KO mice, and then social dominance tests were performed.

Starting 25 days after the virus injection, it was found that the CRTC3 KO mice showed changes in social ranking as their winning points increased in the tube dominance test (FIG. 8A). In particular, it was identified that 30 days after the virus injection, social ranking was reversed among the mice, and the CRTC3 KO mice occupied higher social ranking than the WT mice (FIG. 8A).

Even in the warm spot test, which is another type of social dominance test, it was identified that the CRTC3 KO mice exhibited an increase in the time they occupied the warm spot after being injected with the virus so that AREG is expressed in astrocytes (FIG. 8B).

The results of Example 10 show that inducing expression of AREG specifically in astrocytes in the CRTC3 KO mice with low social dominance ranking can cause an increase in their social dominance ranking, which is consistent with the results of Example 9 verifying that direct administration of AREG protein or the EGF domain of AREG to the mouse brain causes changes in social dominance ranking of the mice.

**Example 11. Identification of functional activation of brain by administration of AREG-EGF**

**Example 11.1. Brain fMRI results in wild-type rats**

To identify which regions of the brain are affected by administration of AREG-EGF, fMRI was performed on wild-type rats injected with AREG-EGF. The rats having received PBS as a control and the rats having received AREG-EGF were imaged with fMRI one month later, in which interaction signals between various regions in the brain were checked. Specifically, the rats, which are SD (Spraque Dawley) rat species, were eight, 5 to 6 week old male rats purchased from Orient Bio. AREG-EGF was administered to the rats by intracerebroventricular injection (ICV) on Weeks 6 to 7 after the purchase, and fMRI imaging was performed 21 days after the administration. The fMRI imaging was performed in such a way that first imaging was performed on an anesthetized rat under normal conditions (pre-state), and then fMRI re-imaging was performed on the same rat by placing cotton soaked in the urine of another rat, which it had never encountered, so that the rat being imaged can sniff the cotton (post-state). For rodents, social responses such as social identification occur through urine marking done by the other individuals. Thus, urine from an unfamiliar rat was used to elicit social responses. For specific methods of administering AREG-EGF and performing MRI analysis, see Experimental Examples 2.1 and 3.

From the experimental results, it was identified that the signal was increased between prefrontal cortex (PFC) and parietal cortex (PC) (FIG. 9A, left). The results obtained by identifying changes in differences among the regions in the brain are shown on the right side of FIG. 9A. In a case where comparison was performed only for the PFC and PC regions using the average z-transformed correlation coefficient (z-CorrCoef; Fisher z-transformed correlation coefficient), the group having received AREG-EGF showed significantly high activity as compared with the group having received PBS (FIG. 9B).

The respective regions with changes (decreased or increased) in the brain, which were identified by the fMRI experimental results, are shown in Table 7 (see Table 1 for the abbreviations).

[Table 7]

| Decreased | | Increased | |
|---|---|---|---|
| Region # 1 | Region #2 | Region #1 | Region #2 |
| Cg1(24b) | Cg2(24a) | PrL | Hb |
| Cg1(24b) | Cg1(24b') | IL | MD |
| Cg1(24b) | PtA | IL | DRN |
| Cg2(24a) | PtA | Cg1(24b') | Hb |
| Cg1(24b') | PtA | Cg1(24b') | CPu |
| Cg1(24b') | PPC | Cg2(24a') | VTA |
| Cg2(24a') | PtA | PtA | NAc |
| Cg2(24a') | PPC | PtA | CPu |
| PtA | PtA | PtA | Amyg |

(continued)

| Decreased | | Increased | |
|---|---|---|---|
| Region # 1 | Region #2 | Region #1 | Region #2 |
| PtA | PPC | PtA | Hypo |
| PPC | HPC | PPC | CPu |
| CPu | Amyg | NAc | Hypo |
| Hypo | DRN | Hb | VTA |
| | | Hb | Hypo |

Next, to identify the spatial location of resting-state functional connectivity (rsFC) regulated by administration of AREG-EGF in PFC a seed-correlation spatial map was generated (FIG. 9C). The Ca1/2 (24b'/24a') region in the brain refers to the cingulate cortex (Cg1, Cg2) and the posterior part (24b' and 24a'). In the statistical map, for rsFC, a significantly enhanced region was observed in PC (LPtA and MPtA) as well as motor (M1), somatosensory (S1DZ, S1BF, and S1Tr), and visual (V1B) cortices (FIG. 9C).

**Example 11.2. Brain fMRI results in mice**

To identify consistency with the fMRI results obtained following administration of AREG in the wild-type rats, fMRI was performed on a CRTC3 KO mouse group. Based on the results identified in Example 11.1, that is, that rsFC is activated in the PFC and PC regions after administration of AREG in the rats, comparison of rsFC was also performed in the CRTC3 KO mouse group.
As a result, as compared with the WT mice, the CRTC3 KO mice showed changes in rsFC in various regions of the brain (FIG. 10A). The CRTC3 KO mice also showed significantly reduced rsFC between PFC and PC that are the regions identified in the rats (FIG. 10B; **p = 0.0099). Seed-based statistical map analysis also verified differences between the WT and CRTC3 KO mouse groups in PFC, PC, and several other regions (left panel of FIG. 10E (Pre-treatment column)). These results demonstrate that CRTC3 plays an important role in connectivity between PC and PFC regions in the mouse brain.
In addition, to identify whether administration of AREG causes changes in rsFC between PC and PFC regions of the CRTC3 KO mice as in the rats, an experiment using the AAV5-GFAP-AREG gene expression system was performed. Specifically, an AAV virus that specifically expresses AREG in astrocytes was injected into the mouse ventricle. As a result of performing fMRI analysis before and after the virus injection, it was found that rsFC, which had been reduced in the CRTC3 KO mice, was restored to a level similar to that of the WT mouse group after injection of the AREG virus (FIGS. 10C and 10D; p = 0.4751). These results could also be clearly identified in the seed-based statistical map analysis (middle panel of FIG. 10E (Post-treatment column)). It was found that differences in rsFC were observed in several regions of the brain between the WT and CRTC3 KO mouse groups before administration of the AREG virus, whereas no difference in rsFC was observed between the WT and CRTC3 KO mouse groups after administration of the virus. These results demonstrate that rsFC between the PC and PFC regions, which was reduced due to CRTC3, can be restored by AREG produced in astrocytes.
The results of Examples 11.1 and 11.2 show that functional activation occurs between PFC and PC of the brain by AREG in a resting state.

**Example 12. Identification of cross-species sequence conservation of EGF and HB domains of AREG**

Like other EGF family growth factors, the precursor of AREG (pro-AREG) consists of EGF domain, a hydrophobic transmembrane region, and a hydrophilic cytoplasmic C-terminus. In addition, AREG contains a heparin binding domain similar to HB-EGF. During cleavage and ectodomain shedding, hydrophilic N-terminal processing at the plasma membrane causes the pro-AREG to be released as a mature protein. In the present example, for the amino acid sequence of AREG, AREG sequences from various species were aligned and compared.
As a result, EGF domain and HB domain were conserved in most AREGs (FIG. 11A). In addition, it was identified that the sequence of each domain of AREG is highly conserved in humans *(Homo sapiens)* and mice *(Mus musculus)* (FIG. 11B).
The results of Example 12 show that the EGF and HB domains of AREG are conserved in various species.
Summarizing the results of Examples 5 to 12, it was identified that the CRTC3 KO mice had very low social dominance ranking as compared with the WT mice, and the molecule, which reverses social inferiority of the CRTC3 KO mice, was amphiregulin (AREG), in particular, the EGF domain of AREG, with AREG being secreted by astrocytes in the brain. These results show that changes in social dominance can be achieved by regulating AREG. Therefore, by applying the newly discovered facts as described above, amphiregulin can be used to prevent, treat, or ameliorate various diseases or conditions caused by decreased social dominance or social inferiority, which results from deficiency of CRTC3, such as chronic inflammation, self-harming, suicidal ideation, anti-social personality disorder, aggressive personality, chronic stress, anxiety neurosis, drug intoxication or mental or behavioral disorder caused by drug intoxication, schizophrenia, mood disorder, mania, depressive disorder, bipolar disorder, fragile X syndrome, autism spectrum disorder, and autism.

## III. Materials and Methods

### Experimental Example 1. Materials and methods for in vitro experiments

### Experimental Example 1.1. Culture and isolation of cells

The cells, which are used in qRT-PCR and microarray experiments for measurement of expression levels of CRTC3 and AREG and transcript profiling depending on cell types, were prepared by the following method.

To culture primary cortical neurons, brain cortex was obtained from the embryonic day 16 (E16) mouse brain as described in the existing literature (see Seibenhener ML, Wooten MW. Isolation and culture of hippocampal neurons from prenatal mice. J Vis Exp. 2012 Jul 26;(65):3634). To remove the meninges from the cortex, the cortex was dissected in cold Hank's Balanced Salt Solution (HBSS) free of calcium and magnesium. The dissected tissue was incubated with 0.05% trypsin-EDTA at 37°C for 15 minutes, and the tube containing the tissue was inverted every 5 minutes. After the incubation, trypsin was inactivated with DMEM containing 20% fetal bovine serum (FBS) and centrifuged at 700 rpm for 15 minutes. The resulting pellet was gently resuspended using a Pasteur pipette. The cells were dissociated in neurobasal medium supplemented with B-27 supplement (Gibco, Life Technologies/Thermo Fisher Scientific, USA) and aliquoted on plates to have an appropriate cell density. The plate was coated with poly-D-lysine (PDL) (Sigma-Aldrich, USA) and laminin (Life Technologies/Thermo Fisher Scientific, USA) and used. Neurons were kept in a humidified environment with 5% $CO_2$ at 37°C.

For culture of glial cells, cerebral cortex was prepared from the mouse pups 1 to 3 days after birth (see Lee J, Kim DE, Griffin P, Sheehan PW, Kim DH, Musiek ES, Yoon SY. Inhibition of REV-ERBs stimulates microglial amyloid-beta clearance and reduces amyloid plaque deposition in the 5XFAD mouse model of Alzheimer's disease. Aging Cell. 2020 Feb;19(2):e13078). As with the culture of primary neuron cells, the dissected tissue was treated with trypsin-EDTA for 15 minutes at 37°C. The cells were centrifuged at 700 rpm for 5 minutes and resuspended in complete medium containing GM-CSF (5 ng/ml). The resuspended cells were plated in 75T flasks and incubated at 37°C for 10 days.

To isolate primary microglia, the flask was shaken at 225 rpm for 2 hours to collect mixed glial cells. Suspended primary microglial cells were plated on PDL-coated plates. The shake flask was maintained for 16 hours to separate other cells. After shaking overnight, attached primary astrocyte cells were cultured on plates for experiments.

All animal experimental procedures used herein were approved by the Ethics Committee for Animal Experiments of the Asan Institute for Life Sciences.

### Experimental Example 1.2. Preparation of RNA and quantitative real-time PCR (qRT-PCR)

Total RNA was extracted from cells using the NucleSpin RNA kit (ACHEREY-NAGEL, Germany) according to the manufacturer's instructions. RNA concentration was measured using an ND-1000 spectrophotometer (NanoDrop Technologies, USA). Purity and integrity of RNA were assessed using an ND-1000 spectrophotometer (NanoDrop Technologies) and an Agilent 2100 bioanalyzer (Agilent Technologies, USA).

To synthesize cDNA, 0.8 μg of RNA was synthesized using the ReverTra Ace qPCR RT Kit (TOYOBO, Japan) according to the manufacturer's instructions. A total reaction volume of the mixture was brought to 20 μl with iQ SYBR Green Supermix (Bio-Rad, USA) and primers. Quantification was performed using the LightCycler® 480 system (Roche Diagnostics, Netherlands) according to the manufacturer's instructions.

All primers used here were synthesized by Cosmogenetech (Seoul, Korea), and the primer sequences are as shown in Table 8.

[Table 8]

| Gene | Orientation of primer | Sequence | Sequence listing |
|---|---|---|---|
| Mouse GAPDH | Forward | 5'-CCATCACCATCTTCCAGGAGCGA-3' | SEQ ID NO: 19 |
| | Reverse | 5'-GGATGACCTTGCCCACAGCCTTG-3' | SEQ ID NO: 20 |
| Mouse CRTC3 | Forward | 5'-CTTCACAGCACCTGGATGAGAG-3' | SEQ ID NO: 21 |
| | Reverse | 5'-TGCTCAGAGCACTCGTGTGAAG-3' | SEQ ID NO: 22 |
| Mouse AREG | Forward | 5'-GCCATTATGCAGCTGCTTTGGACG-3' | SEQ ID NO: 23 |
| | Reverse | 5'-TGTTTTTCTTGGGCTTAATCACCT-3' | SEQ ID NO: 24 |

**Experimental Example 1.3. Microarray analysis**

**Experimental Example 1.3.1. Experimental method**

For transcript profiling, whole transcript expression array (Affymetrix, USA) was performed using the GeneChip Whole Transcript PLUS reagent kit (Affymetrix, USA) according to the manufacturer's protocol. cDNA was synthesized using the GeneChip WT (Whole Transcript) amplification kit as described in the manufacturer's instructions. The sense cDNA was then fragmented with terminal deoxynucleotidyl transferase (TdT) using the GeneChip WT terminal labeling kit and labeled with biotin. Approximately 5.5 $\mu$g of labeled DNA target was hybridized for 16 hours at 45°C using the GeneChip Mouse Gene 2.0 ST Array (Affymetrix, USA). The hybridized array was washed and stained in the GeneChip Fluidics Station 450 and scanned using a GCS3000 scanner (Affymetrix, USA). Signal values were calculated using the GeneChip Command Console software (Affymetrix, USA).

**Experimental Example 1.3.2. Raw data processing and statistical analysis**

Raw data obtained by microarray were automatically extracted by the data extraction protocol provided by GeneChip Command Console software (Affymetrix, USA). CEL files were imported, and then the data were summarized and normalized using the robust multi-array average (RMA) method implemented in Expression Console (EC) software (Affymetrix, USA). The results were output through gene-level RMA analysis, and differentially expressed gene (DEG) analysis was performed.

Comparative analysis between the test and control samples was performed using fold change. For the DEG set, hierarchical cluster analysis was performed using complete linkage and Euclidean distance as measures of similarity. Gene set enrichment analysis and functional annotation analysis of significant probe lists were performed using Gene Ontology (http://geneontology.org/) and KEGG (http://kegg.jp).

All statistical tests and visualizations for DEGs were performed using the R statistical language (v.3.1.2, www.r-project.org).

**Experimental Example 1.4. Immunocytochemistry**

Immunocytochemistry was performed as follows. First, a 12 mm coverslip (Marienfeld, Germany) coated with PDL was placed in a 24-well plate, and cells were plated thereon. Then, the cells were fixed with 4% paraformaldehyde (Biosesang, Korea) for 10 minutes. The cells were washed three times with PBS, and then subjected to permeabilization with PBS containing 0.1% Triton X-100 for 5 minutes at room temperature. The cells were washed again three times and blocked with PBS containing 5% bovine serum albumin (BSA) at 37°C for 30 minutes. The cells were washed three times and incubated with mouse anti-GFAP antibody (Chemicon-Millipore, USA) overnight at 4°C. Subsequently, the cells were washed five times and incubated with secondary antibodies (Invitrogen, USA). Then, each sample was mounted using a fluorescence mounting solution (DAKO, USA) for imaging.

**Experimental Example 1.5. Immunohistochemistry**

Immunohistochemistry was performed as follows. First, the brain was removed from mice, fixed with 4% paraformaldehyde (Biosesang, Korea) at 4°C for 24 hours, and then washed with PBS. For cryosection, each sample was placed in PBS containing 30% sucrose and incubated at 4°C for 24 hours. The sample was embedded in OCT compound (Tissue-Tek, Sakura Finetek, USA) and snap-frozen on dry ice kept at -80°C until sectioning. The frozen sample was then sectioned to a thickness of 30 $\mu$m at -25°C using Leica CM 1860. The obtained sections were washed three times for 5 minutes with PBS, and blocked with PBS containing 1% normal goat serum (NGS) and 0.3% Triton X-100 for 30 minutes. Primary antibodies were diluted with blocking buffer and used, for which treatment was performed overnight at 4°C. The primary antibodies used were as follows: rabbit anti-CRTC3 antibody (Abcam, USA), mouse anti-GFAP antibody (Chemicon-Millipore, USA), rabbit anti-s100$\beta$ antibody (Abcam, USA), mouse anti-NeuN antibody (Abcam, USA), goat anti-Iba1 antibody (Abcam, USA). Subsequently, washing was performed five times with PBS, and then treatment with Alexa Fluor 488 antibody (Invitrogen, USA), biotinylated anti-rabbit IgG antibody (Vector Laboratories, USA), and Alexa Flour 594 conjugated streptavidin (Invitrogen, USA) was performed at room temperature for 60 minutes. For imaging, each sample was mounted using a fluorescence mounting solution (DAKO, USA).

**Experimental Example 1.6. Western blotting**

For extraction of total protein, cells were lysed on ice with PRO-PREP™ reagent (Intron Biotechnology, Korea)-based lysis buffer for 15 minutes and centrifuged to remove cell debris. The concentration of protein samples was determined using the Bradford assay.

For Western blotting analysis, equal amounts of the protein were mixed with a sample buffer (62.5 mM Tris-HCl [pH 6.8], 1% [w/v] SDS, 2.5% [v/v] glycerol, 0.5% [v/v] $\beta$-mercaptoethanol, and bromophenol blue), boiled at 100°C for 5 minutes, and kept at -20°C until use. The protein was separated by sodium dodecyl sulfate-polyacrylamide gel electrophoresis (SDS-PAGE) under a constant voltage (100 V), and then transferred on a polyvinylidene difluoride membrane (PVDF membrane; pore size, 0.2 mm; BioRad, USA) at 100 V for 1.5 hours. After incubation for 1 hour in PBST buffer (with 0.1% [v/v] Tween-20) containing 2% (w/v) BSA and 2% (v/v) normal horse serum (NHS), blot (the PVDF membrane) was incubated with primary antibody overnight at 4°C. The blot was then washed with PBST buffer and incubated with horseradish peroxidase (HRP) conjugated anti-IgG antibody (1:5,000; Pierce, USA). Then, the blot was treated with enhanced chemiluminescence (ECL) reagent (Amersham, USA) and visualized on X-ray film. The primary antibodies used for Western blotting were as follows: rabbit anti-CRTC3 antibody (1:1,000; Cell Signaling, USA), rabbit anti-p-STAT3 antibody (1:500, Cell Signaling, USA), mouse anti-STAT3 antibody (1:2000, Cell Signaling, USA), rabbit anti-p-GluA1(p831) antibody (1:2000; Millipore, USA), rabbit anti-GluA1(p831) antibody (1:2000; Millipore, USA), mouse anti-$\beta$-actin antibody (Sigma, USA). The band intensity was measured and analyzed using ImageJ (Java-based image processing and analysis; National Institutes of Health, USA).

**Experimental Example 2. Surgical method on experimental animals**

**Experimental Example 2.1. Osmotic pump implantation**

AREG, AREG-EGF, AREG-HB, and mouse EGF proteins were dissolved in PBS and injected. The dissolved protein was filled into a micro-osmotic pump (Model 1004, Alzet, France) and allowed to infuse at a flow rate of 0.11 $\mu$l/hr for 25 days. The protein-filled micro-osmotic pump was connected to the cannula via a 4 mm long vinyl tube (inner diameter of 0.69 mm) (Brain Infusion Kit 3, Alzet, France). The completed brain infusion pump was placed in a conical tube filled with PBS, primed at 37°C for 48 hours, and then implanted subcutaneously in a mouse.

Implantation of the brain infusion pump was performed using a stereotaxic apparatus after anesthetizing the mouse with isoflurane (induction: 5%, mask: 2%). The cannula was implanted in the right ventricle (AP [anteroposterior] = -0.3 mm, ML [mediolateral] = 1 mm, based on bregma), and the osmotic pump was implanted in the back of the mouse. At the end of the transplant surgery, the mice were subcutaneously administered ketoprofen (8 mg/kg) as an anti-pain drug. The next day, the ketoprofen administration was repeated once more. To allow for recovery, the mice, which had undergone the transplant surgery, were not subjected to the tube dominance test for 7 days.

For fMRI analysis, a micro-osmotic pump (Model 2004, Alzet, France) implanted in each rat was filled with AREG-EGF and PBS, and allowed to infuse at a flow rate of 0.25 μl/hr for 28 days. The cannula was replaced with a 28 gauge PEEK (polyether ether ketone) cannula. This cannula was implanted into the lateral ventricle of a 5-6 week old rat using a stereotaxic device (based on bregma, AP = -1.2 mm, ML = -2 mm, DV [dorsoventral] = 5 mm). fMRI imaging was conducted 21 days after surgery. fMRI imaging was performed in a basic state (normal condition) after anesthesia of a rat (pre-state), and then performed on the same rat by placing cotton soaked in the urine of another rat, which it had never encountered, so that the rat being imaged can sniff the cotton (post-state). Rats were sacrificed the day after all fMRI imaging was performed.

fMRI analysis in mice was also conducted in the same manner as in rats. Specifically, fMRI imaging was performed a week before virus injection into mice (pre-injection), behavioral tests were performed after the virus injection, and then fMRI imaging was performed at Week 4 to 5 (post-injection). Mice were sacrificed 7 days after completion of fMRI imaging.

**Experimental Example 2.2. Virus stereotaxic injection**

Production of the AAV5-GFAP-mAREG SP_94_191 construct, which is used for expression of AREG protein in astrocytes of the brain, and the AAV5-GFAP-mAREG 94-191 virus obtained using the same was commissioned to VectorBuilder. The nucleotide sequence of mAREG 94-191 in the construct is shown in Table 9:

[Table 9]

| Item | Nucleotide sequence | Sequence listing |
|---|---|---|
| mAREG 94-191 | ATGAGAACTCCGCTGCTACCGCTGGCGCGCTC AGTGCTGTTGCTGCTGGTCTTAGGCTCAGGCCA TTATGCAGCTGCTTCAGTCAGAGTTGAACAGGT GATTAAGCCCAAGAAAAACAAGACAGAAGGA GAAAGTCTACAGAAAAACCCAAAAGGAAGA AAAGGGAGGCAAAAATGGAAAAGGCAGAAG GAATAAGAAGAAAAAGAATCCATGCACTGCCA AGTTTCAGAACTTTTGCATTCATGGCGAATGCA GATACATCGAGAACCTGGAGGTGGTGACATGC AATTGTCATCAAGATTACTTTGGTGAACGGTGT GGAGAAAAATCCATGAAGACTCACAGCGAGGA TGACAAGGACCTATCCAAGTACCCATACGATGT TCCAGATTACGCTTAG | SEQ ID NO: 25 |

The produced virus was injected into the right ventricle of each mouse at an amount of $1 \times 10^{11}$ genome copies (gc) per hemisphere. Specifically, the mouse was anesthetized and fixed to a stereotaxic device, and the location of AP = -0.3 mm, ML = -1.0 mm, and DV = -3.0 mm was marked based on bregma of the mouse skull. A hole was drilled at the indicated location, and the virus was injected at a rate of 0.4 μl/min using a Hamilton syringe. About 5 minutes after the injection, the syringe was slowly removed over 1 minute. The mice having received the virus injection had a recovery period of about a week and then were subjected to behavioral tests.

**Experimental Example 3. Collection and analysis of MRI data**

**Experimental Example 3.1. Collection of MRI data**

MRI was performed using a 7.0 T Bruker PharmaScan 70/16 MRI system (Bruker BioSpin, Germany) with the Para Vision 6.0.1 software in the configuration involving a 72-mm transmit volume coil and a rat brain surface receiver coil. Rats were anesthetized with 50 mg/kg zolazepam-tiletamine combination (Zoletil®, Virbac, Australia) and 12.5 mg/kg Rompun (Bielkorea, Korea). For mice, 0.05 mg/kg of medetomidine was used as an anesthetic, and 0.25 mg/kg of atipamezole was injected to facilitate anesthetic recovery after performing MRI.

While monitoring the animal's respiration, the animal's body temperature was consistently maintained at 37.5 $\pm$ 0.5°C using a circulating water tank system (CW-05G constant temperature circulating water tank; Midwest Scientific, USA).

A single-shot gradient-echo echo-planar imaging (GE-EPI) sequence was used with the following parameters: repetition time (TR) = 1000 ms; echo time (TE) = 20.3 ms; matrix size = 64X64; field of view (FOV) = 25X25 mm$^2$; number of slices = 23; slice thickness = 1 mm; slice spacing = 0 mm; flip angle = 45°, 300 volumes per scan.

In addition, anatomical images were acquired by rapid imaging using the Rapid Imaging with Refocused Echoes (RARE) sequence with the following parameters: repetition time (TR) = 5250 ms; echo time (TE) = 66 ms; matrix size = 256X256; field of view (FOV) = 25X25 mm$^2$; number of slices = 23; slice thickness = 1 mm; slice spacing = 0 mm; number of excitations (NEX) = 2.

**Experimental Example 3.2. Collection of resting state fMRI (rsfMRI) data**

All data from rsfMRI were processed using AFNI (Analysis of Functional NeuroImages; ver.19.2.23, National Institutes of Health, U.S.A., see PMID: 8812068, 9430344, and 9673664) and in-house code of MATLAB (The MathWorks, Inc., USA). Those generally used for human rsfMRI data processing were adjusted to be optimized for rat rsfMRI data processing and used.

The preprocessing pipeline included the following five steps: First, rsfMRI images were co-registered to the EPI template of the rat brain using affine transformation (3dAllineate). Second, the co-registered rsfMRI images were corrected to match the rat's head motions, and the 6-motion parameters were output in ASCII format (3dvolreg). Third, motion-corrected rsfMRI images were obtained through transient spike removal (3dDespike) and polynomial trend removal (3dDetrend). Fourth, the subject's cerebrospinal fluid (CSF)-detrended rsfMRI images were extracted using template mask sets (3dmaskave). Last, the detrended rsfMRI images were obtained using band-pass filtering (0.01 to 0.1 Hz) and spatial smoothing (FWHM = 0.7 mm) with nuisance regression (3dTproject). For evaluation of nuisance signals, 6-motion parameters and CSF signals were evaluated. The CSF mask template of the SIGMA rat brain templates (see PMID: 31836716) was used for a region of interest (ROI) of CSF. The SIGMA CSF mask has regions outside the brain, and thus was modified to strictly include the regions inside the ventricle. Some of the post-registered images were overlaid onto cortical areas in the outer region of the original CSF mask. Removal of the outer region of the CSF mask was performed using the ITK-SNAP program (ver.3.6, see PMID: 16545965). In the present example, a strict ventricular CSF mask was used. The average of time-lapse signals in the strict CSF mask region was estimated from detrended rsfMRI images (3dmaskave).

Collection of rsfMRI data from mice was conducted in the same manner as in rats.

**Experimental Example 3.3. Resting-state functional connectivity (rsFC) analysis**

To measure inter-regional rsFC in the rat brain, seed-based rsFC analysis was performed. A total of 33 ROIs were assessed to evaluate rsFC related to social dominance behavior in rodents. For the average time-course signal in each ROI of the defined anatomical rat brain atlas, an internal ROI mask was used which is defined for the region in question using the SIGMA rat brain atlas and Brain maps 4.0. The rsFC intensity between the rat brain and ROI was estimated using the Pearson's correlation coefficient. Then, the correlation values were subjected to Fisher r-to-z transformation (Fisher r-to-z transformation) to improve normality. Group-level rsFC maps were generated, which were corrected using family-wise error rate (FWER) and false discovery rate (FDR; $p < 0.05$). The seed-based spatial FC map was generated with 1-mm spherical seeds in the posterior cingulate cortex (Cg 1, 24b' and 24a'). The group-level seed map was calculated using the average over each of the seeded spatial FC maps (3dTcorr1D, 3dttest++, and FDR corrected ($p < 0.05$)).

rsFC analysis in mice was performed in the same manner as in rats.

**Experimental Example 4. Statistical analysis**

Data analysis in the examples was performed using GraphPad Prism 5 software. For behavioral tests, data were analyzed using Mann-Whitney test, unpaired t-test, one-way ANOVA post hoc Bonferroni's Multiple Comparison Test, and Tukey's Multiple Comparison Test. The Wilcoxon signed rank test was used to perform comparison between before and after drug administration experiments. For analysis of the bands from Western blotting, t-test or Mann-Whitney test was used for comparison of two groups where appropriate. In a case where variances were not equal as assessed by the Bartlett's test, non-parametric analysis was conducted using the Kruskal-Wallis test and the Dunn's multiple comparison test. The D'Agostino-Pearson omnibus normality test was used to test data normality. A probability of < 5% ($p < 0.05$) was considered significant. All values were expressed as mean $\pm$ standard error of the mean (Mean $\pm$ SEM).

Industrial Applicability

The animal model lacking social dominance according to the present disclosure may be used as an animal model for a disease related to lack or reduction of social dominance, in particular, a disease accompanied by or caused by social defeat stress, and the pharmaceutical composition comprising as an active ingredient an agonist for EGF receptor in the brain according to the present disclosure may be used to prevent and treat a disease related to lack or reduction of social dominance. Thus, the present disclosure is expected to have great industrial applicability.

**Claims**

1. An animal model lacking social dominance, in which expression or activity of CREB-regulated transcription coactivator 3 (CRTC3) gene or protein is suppressed.

2. The animal model of claim 1, wherein the expression or activity of CRTC3 gene or protein is specifically suppressed in the brain.

3. The animal model of claim 1, wherein the expression or activity of CRTC3 gene or protein is specifically suppressed in astrocytes of the brain.

4. The animal model of claim 1, wherein the CRTC3 gene is knocked out or knocked down.

5. The animal model of claim 1, wherein expression or activity of amphiregulin gene or protein is decreased in the brain as compared with a wild-type animal.

6. The animal model of claim 1, wherein the animal model shows no changes in memory-related behavior, sensory-related behavior, or both as compared with a wild-type animal.

7. The animal model of claim 1, wherein the animal model has decreased functional connectivity between prefrontal cortex and parietal cortex in the brain as compared with a wild-type animal.

8. The animal model of claim 1, wherein the animal model is a model for a disease related to lack or reduction of social dominance.

9. The pharmaceutical composition of claim 8, wherein the disease is a disease accompanied by or caused by social defeat stress.

10. The animal model of claim 8, wherein the disease is at least one selected from the group consisting of chronic inflammation, self-harming, suicidal ideation, anti-social personality disorder, aggressive personality, chronic stress, anxiety neurosis, drug intoxication or mental or behavioral disorder caused by drug intoxication, schizophrenia, mood disorder, mania, depressive disorder, bipolar disorder, fragile X syndrome, autism spectrum disorder, and autism.

11. The animal model of claim 1, wherein the animal is selected from the group consisting of mouse, hamster, rat, guinea pig, monkey, dog, cat, rabbit, cow, sheep, pig, and goat.

12. A method of producing an animal model lacking social dominance, comprising suppressing expression or activity of CREB-regulated transcription coactivator 3 (CRTC3) gene or protein.

13. The method of claim 12, wherein the suppressing comprises knocking out or knocking down the CRTC3 gene.

14. A method for screening a therapeutic agent for a psychiatric disease, comprising administering a candidate drug for treatment of a psychiatric disease to the animal model lacking social dominance of any one of claims 1 to 11; and determining whether social dominance of the animal model is improved.

15. The method of claim 14, wherein the psychiatric disease is accompanied by symptoms of lack or reduction of social dominance.

16. The method of claim 14, wherein the determining is performed by a behavioral test for evaluating social dominance, electroencephalogram (EEG) analysis, brain MRI analysis, measurement of expression or activity level of amphiregulin in a biological sample derived from the animal model, or a combination thereof.

17. A pharmaceutical composition for preventing or treating a disease related to lack or reduction of social dominance, comprising as an active ingredient an agonist for epidermal growth factor (EGF) receptor in the brain.

18. The pharmaceutical composition of claim 17, wherein the agonist for EGF receptor comprises amphiregulin, a fragment thereof, or a nucleic acid encoding the same.

19. The pharmaceutical composition of claim 18, wherein the amphiregulin or the fragment thereof comprises EGF domain of amphiregulin.

20. The pharmaceutical composition of claim 18, wherein the amphiregulin comprises the amino acid sequence represented by SEQ ID NO: 8.

21. The pharmaceutical composition of claim 19, wherein the EGF domain of amphiregulin comprises the amino acid sequence represented by SEQ ID NO: 10.

22. The pharmaceutical composition of claim 17, wherein the disease is caused by impaired expression or decreased activity of CREB-regulated transcription coactivator 3 (CRTC3).

23. The pharmaceutical composition of claim 17, wherein the disease is accompanied by or caused by social defeat stress.

24. The pharmaceutical composition of claim 17, wherein the disease is at least one selected from the group consisting of chronic inflammation, self-harming, suicidal ideation, anti-social personality disorder, aggressive personality, chronic stress, anxiety neurosis, drug intoxication or mental or behavioral disorder caused by drug intoxication, schizophrenia, mood disorder, mania, depressive disorder, bipolar disorder, fragile X syndrome, autism spectrum disorder, and autism.

25. The pharmaceutical composition of claim 17, wherein the composition is administered by intracerebral injection or intracerebroventricular injection (ICV).

26. A method for preventing or treating a disease related to lack or reduction of social dominance, comprising administering to a subject the pharmaceutical composition of any one of claims 17 to 25.

[FIG. 1A]

[FIG. 1B]

[FIG. 1C]

[FIG. 2A]

WT          CRTC3 KO

[FIG. 2B]

Tube test

[FIG. 2C]

Push-initiated

[FIG. 2D]

Push-back

[FIG. 2E]

[FIG. 2F]

[FIG. 2G]

Familiar group

Unfamiliar group

[FIG. 2H]

weight matched

[FIG. 2I]

## Female (same cages)

[FIG. 2J]

Female (different cages)

[FIG. 2K]

[FIG. 2L]

[FIG. 2M]

[FIG. 3A]

Y-maze

[FIG. 3B]

Water maze

[FIG. 3C]

Olfactory preference test

[FIG. 3D]

Novel obeject recognition

[FIG. 4A]

[FIG. 4B]

[FIG. 5A]

[FIG. 5B]

Primary actrocyte

[FIG. 5C]

Primary astrocyte - FSK

[FIG. 5D]

[FIG. 5E]

[FIG. 5F]

[FIG. 6A]

[FIG. 6B]

[FIG. 7A]

WT, CRTC3 KO group-AREG infusion

[FIG. 7B]

Warm spot

[FIG. 7C]

C57BL/6 group - AREG infusion

[FIG. 7D]

C57BL/6 group - AREG-EGF infusion

[FIG. 7E]

WT, CRTC3 KO group - AREG-EGF infusion

[FIG. 7F]

[FIG. 8A]

[FIG. 8B]

[FIG. 9A]

[FIG. 9B]

[FIG. 9C]

[FIG. 10A]

[FIG. 10B]

[FIG. 10C]

[FIG. 10D]

[FIG. 10E]

[FIG. 11A]

EP 4 368 196 A1

[FIG. 11B]

# INTERNATIONAL SEARCH REPORT

| International application No. |
|---|
| **PCT/KR2022/009893** |

| **A. CLASSIFICATION OF SUBJECT MATTER** |
|---|
| **A61K 38/18**(2006.01)i; **A61P 25/30**(2006.01)i; **A61P 25/18**(2006.01)i; **A61P 25/24**(2006.01)i; **A23L 33/17**(2016.01)i; **C12Q 1/6883**(2018.01)i; **G01N 33/68**(2006.01)i; **G01N 33/74**(2006.01)i; **A61B 5/00**(2006.01)i |
| According to International Patent Classification (IPC) or to both national classification and IPC |

| **B. FIELDS SEARCHED** |
|---|
| Minimum documentation searched (classification system followed by classification symbols) |
| A61K 38/18(2006.01); C07K 14/435(2006.01); C12Q 1/68(2006.01) |
| Documentation searched other than minimum documentation to the extent that such documents are included in the fields searched |
| Korean utility models and applications for utility models: IPC as above<br>Japanese utility models and applications for utility models: IPC as above |
| Electronic data base consulted during the international search (name of data base and, where practicable, search terms used) |
| eKOMPASS (KIPO internal) & keywords: CRTC3(CREB-regulated transcription coactivator 3), 사회적 우세성(social dominance), 성상교세포(astrocyte), 암피레귤린(amphiregulin, AREG), 사회적 패배 스트레스(social defeat stress), EGFR(epidermal growth factor receptor), 불안신경증(anxiety), 조현병(schizophrenia), 우울증(depressive disorder), 자폐 스펙트럼 장애(autism spectrum disorder) |

| **C. DOCUMENTS CONSIDERED TO BE RELEVANT** | | |
|---|---|---|
| Category* | Citation of document, with indication, where appropriate, of the relevant passages | Relevant to claim No. |
| A | KR 10-2017-0036929 A (UNIVERSITY OF ULSAN FOUNDATION FOR INDUSTRY COOPERATION) 04 April 2017 (2017-04-04)<br>See paragraphs [0031] and [0055]-[0058]. | 1-25 |
| A | KR 10-2007-0084498 A (NOVARTIS AG) 24 August 2007 (2007-08-24)<br>See claims 22, 23, 26 and 28. | 1-25 |
| A | JUREK, B. et al. Oxytocin regulates stress-induced Crf gene transcription through CREB-regulated transcription coactivator 3. Journal of Neuroscience. 2015, vol. 35, no. 35, pp. 12248-12260.<br>See abstract. | 1-25 |
| A | OSTOJIC, J. et al. Transcriptional co-activator regulates melanocyte differentiation and oncogenesis by integrating cAMP and MAPK/ERK pathways. Cell Reports. 2021, vol. 35, no. 7, article no. 109136, inner pp. 1-11 (publication date: 18 May 2021).<br>See abstract; and inner pages 1 and 2. | 1-25 |

☑ Further documents are listed in the continuation of Box C.　　☑ See patent family annex.

| * | Special categories of cited documents: |
|---|---|
| "A" | document defining the general state of the art which is not considered to be of particular relevance |
| "D" | document cited by the applicant in the international application |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
|---|---|
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
|---|---|
| **19 October 2022** | **19 October 2022** |

| Name and mailing address of the ISA/KR | Authorized officer |
|---|---|
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

## INTERNATIONAL SEARCH REPORT

International application No.

**PCT/KR2022/009893**

**C.    DOCUMENTS CONSIDERED TO BE RELEVANT**

| Category* | Citation of document, with indication, where appropriate, of the relevant passages | Relevant to claim No. |
|---|---|---|
| A | ESCOUBAS, C. C. et al. Deregulation of CRTCs in aging and age-related disease risk. Trends in Genetics. 2017, vol. 33, no. 5, pp. 303-321.<br>    See abstract; and pages 303-316. | 1-25 |

Form PCT/ISA/210 (second sheet) (July 2022)

**INTERNATIONAL SEARCH REPORT**

International application No.

**PCT/KR2022/009893**

| Box No. I | Nucleotide and/or amino acid sequence(s) (Continuation of item 1.c of the first sheet) |
|---|---|

1. With regard to any nucleotide and/or amino acid sequence disclosed in the international application, the international search was carried out on the basis of a sequence listing:

   a. ☑ forming part of the international application as filed.

   b. ☐ furnished subsequent to the international filing date for the purposes of international search (Rule 13*ter*.1(a)),

      ☐ accompanied by a statement to the effect that the sequence listing does not go beyond the disclosure in the international application as filed.

2. ☐ With regard to any nucleotide and/or amino acid sequence disclosed in the international application, this report has been established to the extent that a meaningful search could be carried out without a WIPO Standard ST.26 compliant sequence listing.

3. Additional comments:

Form PCT/ISA/210 (continuation of first sheet) (July 2022)

## INTERNATIONAL SEARCH REPORT

International application No.

**PCT/KR2022/009893**

| Box No. II | Observations where certain claims were found unsearchable (Continuation of item 2 of first sheet) |
|---|---|

This international search report has not been established in respect of certain claims under Article 17(2)(a) for the following reasons:

1. ☑ Claims Nos.: **26**
because they relate to subject matter not required to be searched by this Authority, namely:

Claim 26 pertains to a method for treatment of the human body by surgery or therapy, as well as a diagnostic method (PCT Article 17(2)(a)(i) and PCT Rule 39.1(iv)).

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

Form PCT/ISA/210 (continuation of first sheet) (July 2022)

**INTERNATIONAL SEARCH REPORT**
Information on patent family members

| International application No. |
|---|
| **PCT/KR2022/009893** |

| Patent document cited in search report | | | Publication date (day/month/year) | Patent family member(s) | | | Publication date (day/month/year) |
|---|---|---|---|---|---|---|---|
| KR | 10-2017-0036929 | A | 04 April 2017 | KR | 10-1854962 | B1 | 08 May 2018 |
| KR | 10-2007-0084498 | A | 24 August 2007 | AU | 2005-336514 | A1 | 17 May 2007 |
| | | | | AU | 2005-336514 | A8 | 11 December 2008 |
| | | | | AU | 2005-336514 | A8 | 12 April 2007 |
| | | | | BR | PI0517021 | A | 30 September 2008 |
| | | | | CA | 2589430 | A1 | 12 April 2007 |
| | | | | CN | 101090912 | A | 19 December 2007 |
| | | | | EP | 1807447 | A2 | 18 July 2007 |
| | | | | JP | 2008-517627 | A | 29 May 2008 |
| | | | | MX | 2007004968 | A | 15 June 2007 |
| | | | | RU | 2007119313 | A | 27 November 2008 |
| | | | | US | 2009-0202565 | A1 | 13 August 2009 |
| | | | | WO | 2007-040550 | A2 | 12 April 2007 |
| | | | | WO | 2007-040550 | A3 | 28 June 2007 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- KR 1020210089195

**Non-patent literature cited in the description**

- **ANACKER AM ; SMITH ML ; RYABININ AE.** Establishment of stable dominance interactions in prairie vole peers: relationships with alcohol drinking and activation of the paraventricular nucleus of the hypothalamus. *Soc Neurosci,* 2014, vol. 9 (5), 484-94
- **ZHOU Y ; KAISER T ; MONTEIRO P ; ZHANG SAN-JANA N ; ZHOU Y ; ZHANG M ; ZHANG F ; FU Z ; FENG G.** Mice with Shank3 Mutations Associated with ASD and Schizophrenia Display Both Shared and Distinct Defects. *Neuron,* 06 January 2016, vol. 89 (1), 147-62
- **WALLEN-MACKENZIE A ; NORDENANKAR K ; FEJGIN K ; LAGERSTRφM MC ; EMILSSON L ; FREDRIKSSON R ; WASS C ; ANDERSSON D ; EGECIOGLU E ; ANDERSSON M.** Restricted cortical and amygdaloid removal of vesicular glutamate transporter 2 in preadolescent mice impacts dopaminergic activity and neuronal circuitry of higher brain function. *J Neurosci.,* 18 February 2009, vol. 29 (7), 2238-51
- **YANG CR ; BAI YY ; RUAN CS ; ZHOU HF ; LIU D ; WANG XF ; SHEN LJ ; ZHENG HY ; ZHOU XF.** Enhanced aggressive behavior in a mouse model of depression. *Neurotox Res.,* February 2015, vol. 27 (2), 129-42
- **SAXENA K ; WEBSTER J ; HALLAS-POTTS A ; MACKENZIE R ; SPOONER PA ; THOMSON D ; KIND P ; CHATTARJI S ; MORRIS RGM.** Experiential contributions to social dominance in a rat model of fragile-X syndrome. *Proc Biol Sci.,* 13 June 2018, vol. 285 (1880), 20180294
- **HUANG WH ; WANG DC ; ALLEN WE ; KLOPE M ; HU H ; SHAMLOO M ; LUO L.** Early adolescent Rai1 reactivation reverses transcriptional and social interaction deficits in a mouse model of Smith-Magenis syndrome. *Proc Natl Acad Sci US A.,* 16 October 2018, vol. 115 (42), 10744-10749
- **FUKUDA S ; NISHIDA-FUKUDA H ; NAKAYAMA H ; INOUE H ; HIGASHIYAMA S.** Monoubiquitination of pro-amphiregulin regulates its endocytosis and ectodomain shedding. *Biochem Biophys Res Commun.,* 06 April 2012, vol. 420 (2), 315-20
- **SHOYAB M ; PLOWMAN GD ; MCDONALD VL ; BRADLEY JG ; TODARO GJ.** Structure and function of human amphiregulin: a member of the epidermal growth factor family. *Science,* 24 February 1989, vol. 243 (4894), 1074-6
- **KATO M ; INAZU T ; KAWAI Y ; MASAMURA K ; YOSHIDA M ; TANAKA N ; MIYAMOTO K ; MIYA-MORI I.** Amphiregulin is a potent mitogen for the vascular smooth muscle cell line, A7r5. *Biochem Biophys Res Commun.,* 21 February 2003, vol. 301 (4), 1109-15
- **FALK A ; FRISM J.** Amphiregulin is a mitogen for adult neural stem cells. *J Neurosci Res,* 15 September 2002, vol. 69 (6), 757-62
- **LEA AJ ; AKINYI MY ; NYAKUNDI R ; MARERI P ; NYUNDO F ; KARIUKI T ; ALBERTS SC ; ARCHIE EA ; TUNG J.** Dominance rank-associated gene expression is widespread, sex-specific, and a precursor to high social status in wild male baboons. *Proc Natl Acad Sci U.S.A.,* 26 December 2018, vol. 115 (52), E12163-E12171
- **WOLKE D ; LEREYA ST.** Long-term effects of bullying. *Arch Dis Child.,* September 2015, vol. 100 (9), 879-85
- **MUELLER AS ; ABRUTYN S ; PESCOSOLIDO B ; DIEFENDORF S.** The Social Roots of Suicide: Theorizing How the External Social World Matters to Suicide and Suicide Prevention. *Front Psychol.,* 31 March 2021, vol. 12, 621569
- **HALPERN J ; JUTTE D ; COLBY J ; BOYCE WT.** Social dominance, school bullying, and child health: what are our ethical obligations to the very young?. *Pediatrics,* March 2015, vol. 135 (2), S24-30
- **KARAMIHALEV S ; BRIVIO E ; FLACHSKAMM C ; STOFFEL R ; SCHMIDT MV ; CHEN A.** Social dominance mediates behavioral adaptation to chronic stress in a sex-specific manner. *Elife,* 09 October 2020, vol. 9, e58723
- **J PRICE.** The dominance hierarchy and the evolution of psychiatric disease. *The Lancet.,* vol. 290 (7509), 243-246
- Remington's Pharmaceutical Sciences and U.S. Pharmacopeia: National Formulary. Mack Publishing Company, 1984

- **SONG Y ; ALTAREJOS J ; GOODARZI MO ; IN-OUE H ; GUO X ; BERDEAUX R ; KIM JH ; GOODE J ; IGATA M ; PAZ JC.** CRTC3 links catecholamine signaling to energy balance. *Nature,* 16 December 2010, vol. 468 (7326), 933-9
- **PARK MJ ; SEO BA ; LEE B ; SHIN HS ; KANG MG.** Stress-induced changes in social dominance are scaled by AMPA-type glutamate receptor phosphorylation in the medial prefrontal cortex. *Sci Rep.,* 09 October 2018, vol. 8 (1), 15008
- **KIM JW ; PARK K ; KANG RJ ; GONZALES ELT ; KIM DG ; OH HA ; SEUNG H ; KO MJ ; KWON KJ ; KIM KC.** Pharmacological modulation of AMPA receptor rescues social impairments in animal models of autism. *Neuropsychopharmacology,* January 2019, vol. 44 (2), 314-323
- **SEIBENHENER ML ; WOOTEN MW.** Isolation and culture of hippocampal neurons from prenatal mice. *J Vis Exp.,* 26 July 2012, (65), 3634
- **LEE J ; KIM DE ; GRIFFIN P ; SHEEHAN PW ; KIM DH ; MUSIEK ES ; YOON SY.** Inhibition of REV-ERBs stimulates microglial amyloid-beta clearance and reduces amyloid plaque deposition in the 5XFAD mouse model of Alzheimer's disease. *Aging Cell,* February 2020, vol. 19 (2), e13078